(19) Europäisches Patentamt / European Patent Office / Office européen des brevets

(11) **EP 2 270 698 A1**

(12) **EUROPEAN PATENT APPLICATION**

(43) Date of publication:
**05.01.2011 Bulletin 2011/01**

(51) Int Cl.:
***G06F 19/00*** *(2011.01)*

(21) Application number: **06291901.4**

(22) Date of filing: **11.12.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Avant-Garde Materials Simulation Sarl F-78100 St-Germain-en-Laye (FR)**

(72) Inventor: **Neumann, Marcus A., c/o Avant-Garde Materials 78100 St-Germain-en-Laye (FR)**

(74) Representative: **Betten & Resch Patentanwälte Theatinerstrasse 8 80333 München (DE)**

(54) **Tailor-made force fields for crystal structure prediction**

(57)    A general procedure is presented to derive force field parameters for molecules in the crystalline state on a case by case basis. The force field parameters are fitted to accurate energies and forces generated by means of a hybrid method that combines DFT calculations with an empirical van der Waals correction. The mathematical structure of the force field, the generation of reference data, the choice of the figure of merit, the optimization algorithm and the parameter refinement strategy are discussed in detail.

**Fig. 1**

EP 2 270 698 A1

**Description**

The present invention relates to a method for the prediction of crystal polymorphs for a given molecule as well as to a method for the determination of the parameters of a force field for the calculation of lattice energies and/or energy derivatives for at least one molecular crystal.

A general procedure is presented to derive force field parameters for molecules in the crystalline state on a case by case basis. The force field parameters are fitted to accurate energies and forces generated by means of a hybrid method that combines DFT (density functional theory) calculations with an empirical van der Waals correction. The mathematical structure of the force field, the generation of reference data, the choice of the figure of merit, the optimization algorithm and the parameter refinement strategy are discussed in detail. In an exemplary embodiment of the invention, the approach is applied to a small organic molecule, cyclohexane-1,4-dione. The tailor-made force field obtained for this compound is used to generate low energy crystal packings in all 230 space groups with one molecule per asymmetric unit and the most stable crystal structures are re-optimized in a second step with the hybrid method. The average disagreement between the lattice energies calculated with the tailor-made force field and the hybrid method is found to be 0.033 kcal/mol per atom. The experimental crystal structure is found as the most stable predicted crystal structure both with the tailor-made force field according to the invention and the hybrid method.

The invention furthermore relates to a computer program product comprising computer readable code for enabling a computer to perform the method according to the invention.

## 1. Introduction

Many physical and chemical properties of molecular crystals, such as density, colour, solubility and dissolution rate, strongly depend on the molecular packing in the solid state. The prediction of the experimentally accessible crystal structures therefore is a prerequisite for the computation of important solid state properties from the molecular structure as a starting point. While crystal structure prediction is an essential step towards the distant goal of in silico materials design, one close-by application is the prediction of the most stable crystal polymorph for a given molecule. Especially in the pharmaceuticals industry, formulations based on the crystal polymorph with the lowest free energy are generally preferred, because they provide the best long term stability. However, the stable crystal polymorph may only show up experimentally several years after the first synthesis of a new drug compound because of unfavourable crystallization kinetics. The computational prediction of the stable form would greatly reduce the risk of unpleasant surprises during clinical testing and production scale up.

The important potential applications of crystal structure prediction have triggered a lot of work and the state-of-the-art has been assessed by a series of blind tests organized by the Cambridge Crystallographic Data Centre.[1,2,3] The problem of crystal structure prediction can be roughly divided into two challenges: the generation of all relevant low energy crystal packings and the identification of the experimentally accessible crystal structures from the list of candidate structures. The second issue is generally tackled by lattice energy calculations, assuming that it is the most stable crystal structures that are observed experimentally. The blind tests clearly reveal energy ranking as the main bottleneck for the crystal structure prediction of rigid or slightly flexible molecules. Indeed, many of the experimental crystal structures were actually generated by one or more participants of the CCDC blind tests, but whether or not these structures were selected as one of the three allowed submissions per participant often turned out to be a matter of luck. Indeed, the lattice energy differences between the most stable crystal structures are typically smaller that the accuracy of common methods for lattice energy calculations.

In an attempt to solve the energy ranking problem, we have recently developed a hybrid method[4] that combines DFT calculations by means of the VASP program[5,6,7,8,9] with an empirical van der Waals correction fitted to molecular $C_6$ coefficients and the unit cells of low temperature crystal structures. When initially apply to the energy ranking of crystal packings generated with Accelrys' Polymorph Predictor[10] in the 17 most frequent space groups, the hybrid method performed excellently for a series of 6 small and essentially rigid molecules, namely acetylene, ethylene, ethane, methanol, acetic acid and urea. In 5 out of 6 cases, the experimental crystal structure was found as the most stable predicted crystal structure and for ethylene it came second. The high potential of the hybrid method was confirmed by an additional energy ranking study[11] on the submitted and the experimental crystal structures of the first two CCDC blind tests. Out of 7 considered experimental crystal structures, 6 turned up among the two most stable predicted crystal structures. The remaining ranking imperfections are not necessarily related to the inherent inaccuracy of the hybrid method. Zero-point vibrational energies and entropie effects are currently not taken into account, and the energy ranking may improve further if free energies at the experimental temperatures are calculated rather than lattice energies. In addition, the stable polymorph may not yet have been observed in some cases. For the remainder of this work we will assume that the hybrid method provides the accuracy required for crystal structure prediction.

The hybrid method is too time consuming to be used directly for the generation of crystal structures, since the structure generation algorithm must explore many thousands or even millions of possible packing alternatives. Instead, a fast but less accurate method is required for the purpose of crystal structure generation, and only the best candidates are optimized in a last step with a hybrid method. Force fields are the usual choice for fast lattice energy calculations during the structure generation procedure. A force field in molecular modelling has little to do with the notion of a field in physics. In the area of molecular modelling, a force field is simply a term for a set of mathematical functions and parameters which yield an empirical description of the lattice energy as a function of the atomic coordinates and the lattice parameters. For an easy introduction to empirical force fields we refer the reader to chapter 4 in Ref. 28.

While working on our own crystal structure generation engine, we rapidly realised that the hybrid method provides only a limited solution to the energy ranking problem. Using standard force fields, the number of crystal structures in the energy window between the experimental crystal structure and the most stable predicted crystal structure grows rapidly with the molecular flexibility. As a consequence, it becomes increasingly time consuming to treat a sufficiently large number of crystal structures in the final energy ranking step. With the hybrid method, the final energy ranking is essentially limited to 10-100 crystal structures. The CPU time and memory requirements for the DFT component of the hybrid method rapidly grow with system size. For a crystal structure with a reduced unit cell volume of 3000 $Å^3$, a full crystal structure optimization from a reasonably good starting point takes about 2-4 weeks of CPU time on two state-of-the-art PCs working in parallel. Hence, improvements of the current force field technology remain of vital importance to narrow down the number of crystal structures to be optimized with the hybrid method. In passing we note that, because of the high CPU time requirements involved, the direct use of DFT calculations during structure generation, as recently demonstrated for several inorganic and organic compounds including urea[12], will not become feasible for most organic compounds of industrial interest for many years to come.

Force field parameterization using experimental data and/or ab initio calculations is generally believed to belong to the realm of some enlightened specialists, being far too complicated for every day users of molecular modelling software. Most molecular modelling studies use one of the published force fields such as Dreiding[13], CHARMM[14], Amber[15], Merck Molecular Force Field[16], CVFF[17], COMPASS[18] or UFF[19]. Molecule specific re-parameterization, if carried out at all, is generally limited to the charge model. For the purpose of crystal structure prediction, it is widely admitted that atomic charges should be fitted to the electrostatic potential around the molecule derived from ab initio calculations. It has also been shown that the accuracy of force field calculations can be improved if electrostatic multipoles are used instead of point charges.[20-23]

Compared to the parameterization of other force field parameters, the extraction of atomic point charges and/or electrostatic multipoles from an electronic wave function is relatively straight forward, but the re-parameterization of the charge model only on a molecule by molecule basis suffers from serious methodological shortcomings. In fact, molecular and crystalline equilibrium structures result from a fine balance of many force field terms, including electrostatic interactions and vdW terms as well as bond stretch, angle bend, torsion and inversion terms for covalently bonded atoms. In carefully parameterized force fields, the various terms complement each other. If one type of interaction is re-parameterized, so should be the others. The Coulomb energy between two atoms on neighbouring molecules, for instance, does not only depend on the atomic charges, but also on the close contact distance which is strongly influenced by the repulsive part of the vdW potential. Intermolecular flexibility is another tricky issue. For a correct description of different molecular conformations, the intramolecular Coulomb and vdW interactions must be carefully balanced by torsion and inversion terms. If only the electrostatic terms are modified, this balance is lost.

It is the main effect of the present invention to suggest a paradigm shift. Instead of supplying the user with one or more transferable force fields parameterized once and for all for different classes of compounds, software tools for accuracy sensitive molecular modelling applications such as crystal structure prediction should provide an accurate method for the generation of reliable reference data and a robust procedure for an extensive force field parameter refinement on a molecule by molecule basis using the aforementioned reference data. By applying the hybrid method to crystal structures containing only a small number of molecules or molecular fragments, it is possible to accumulate a large amount of accurate forces and lattice energies, thus enabling the refinement of many independent force field parameters.

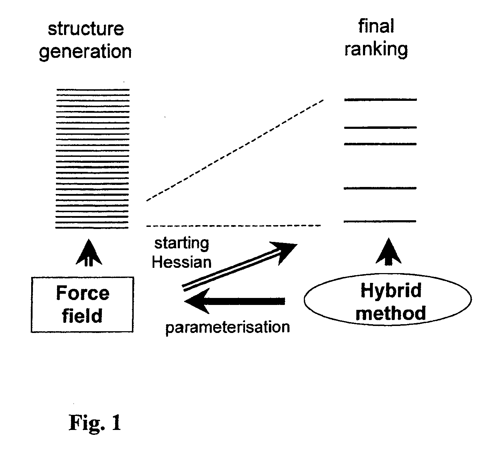

The basic approach for crystal structure prediction according to the invention is summarized in Fig. 1. For a given molecule, the hybrid method is used to parameterise a tailor-made force field. Using this force field, crystal structures are generated and optimized. From the parameterisation step, the accuracy of the force field is roughly known and can be used to delimit a candidate window from which experimentally observable crystal structures may come. All crystal structures in the candidate window are re-ranked and re-optimized with the hybrid method, using a quasi-Newton optimizer and starting Hessians calculated with the tailor-made force field. Alternatively, the size of the candidate window may be calculated on the fly during the final energy ranking with the hybrid method (see eqs. 13-15 in Ref. 4). The accuracy of the tailor made force field affects the CPU time requirements of the final energy ranking in three different ways. With increasing force field accuracy, the size of the energy window and thus the number of structures to optimize decrease. In addition, the equilibrium crystal structures obtained with the force field, which serve as starting points for the final crystal structure optimization, approach the energy minima according to the hybrid method. Furthermore, the accuracy of the starting Hessians improves. From the above comments it is obvious that the quality of the force field parameter refinement has a very strong impact on the CPU time requirements of the final energy ranking step, and spending a significant fraction of the overall CPU time for a crystal structure prediction study on force field refinement is definitely a wise move.

For the present study, we have limited ourselves to a simple mathematical framework for the tailor-made force field involving Coulomb interactions between fixed atomic charges, isotropic van der Waals potentials and essentially uncoupled intramolecular energy terms. This choice was motivated by two different factors. From a practical point of view, a simple mathematic framework can be expected to be good enough for molecules with a zero net charge, if the tailor-made force field is used only for structure generation, but not for the final energy ranking. Moreover, force field parameterization on a molecule by molecule basis is a difficult endeavour because of the large number of parameters involved, and we have preferred to first test our parameter refinement strategy in conjunction with a relatively simple mathematical framework before moving on to more sophisticated energy terms with an increased number of parameters to refine. In this context, it is important to note that the added benefit of more complex electrostatic charge models as explored by several authors[20,21,23,24] is not well characterized, since the re-parameterization of important, interdependent force field parameters is virtually always missing. Despite the fact that our tailor-made force field is based on the same approximations as other common force fields, its energy terms are different in many important respects, because the requirements of a transferable and a tailor-made force field are very dissimilar. In the following, we will frequently compare our own approach to the Dreiding[13] force field, from which we borrow starting values for force field parameters whenever possible.

In addition to a mathematical framework, force field parameter refinement requires reference data, a deviation function (Fig. of merit), a refinement algorithm and a recipe. All these ingredients are described separately in sections 2 to 6. Our hardware setup and some parallelization issues are briefly discussed in section 7, followed by a case study for cyclohexane-1,4-dione in section 8.

Throughout the work described in this document we have used a structure generation engine which for one moderately flexible molecule per asymmetric unit provides a quasi complete list of all low energy crystal packings, searching in all 230 space groups. All algorithms used for the method according to the invention have been implemented and tested in a computer program.

## 2. Mathematical framework

When using a force field, the lattice energy of a crystal is decomposed into several sums over many different energy terms. In the preferred embodiments of the invention we use the following decomposition, although in other embodiments other terms may be added:

$$E_{total}(\vec{x}) = \sum_{Coulomb} E_{Coulomb,i}(\vec{x}) + \sum_{vdW} E_{vdW,i}(\vec{x}) + \sum_{bond} E_{bond,i}(\vec{x}) + \sum_{angle} E_{angle,i}(\vec{x}) + \sum_{torsion} E_{torsion,i}(\vec{x})$$
$$+ \sum_{inversion} E_{inversion,i}(\vec{x}) + \sum_{coupling} E_{coupling,i}(\vec{x}) \tag{0}$$

Here the vector $\vec{x}$ stands for the atomic positions in the unit cell and the lattice parameters. The first two sums named 'Coulomb' and 'vdW' (van der Waals) are double sums that run over all atoms in the unit cell and all atoms that an atom in the unit cell interacts with. Both sums are in principle infinite sums and must be appropriately truncated. The individual energy terms under these sums are described in sections 2.2 and 2.3. The sum named 'bond' runs over all covalent bonds in the unit cell. The bond stretch energy terms are described in subsection 2.5. The sum named 'angle' runs over all angles in the unit cell that are formed by two covalent bonds of the same central atoms. Some of the angles may actually be excluded from the sum, if a corresponding angle bend - inversion coupling term exists. The angle bend terms are described in subsection 2.6. The sum named 'torsion' runs over all non-terminal bonds in the unit cell. The torsion terms are described in subsection 2.7. The sum named 'inversion' runs over all atoms with three covalently bonded neighbours in the unit cell. Some of the inversions may actually be excluded from the sum, if a corresponding angle bend - inversion coupling term exists. The inversion terms are described in subsection 2.8. The sum named 'coupling' runs over special angle bend - inversion coupling terms that may be defined for some atoms with three covalently bonded neighbours. Angle-bend coupling terms are described in subsection 2.9.

Eq. 0 is sometimes called the energy expression. When the lattice energy is to be calculated for a crystal structure, the first thing to do is to set up the energy expression, which involves taking parameters (and a mathematical function) from the force field for every energy term that needs to be considered. Most parameter assignments are based on force field atom types (FFATs). First, each atom in the unit cell is attributed a FFAT, whereby several non-symmetry related atoms may be attributed the same FFAT. Then, for every energy term the FFATs of the involved atoms are compared to the FFATs in lookup tables of the force field. If a match is found, the mathematical function and the parameters that belong to the matched entry in the look up table are used for the energy term under consideration. A force field can be seen as a collection of typing rules and look up tables that allow for the definition of energy terms for specific crystal structures.

Based on this background the invention will be explained in the following with reference to the accompanying drawings in which:

Fig. 1 illustrates an overall strategy for crystal structure prediction;

Figs. 2a to 2d illustrate force field atom types generated automatically for some molecules;

Figs. 3a and 3b illustrate atom indices for the definition of the handedness and the $sp_2$-$sp_2$ rotation criterion, respectively;

Fig. 4 schematically illustrates charge transfer in a simple molecular salt;

Fig. 5 schematically presents atom indices for overall torsions;

Fig. 6 schematically presents atom indices for overall inversions and inversion - angle bend coupling;

Fig. 7 shows an example for grid points for the characterization of the electrostatic potential;

Figs. 8a to 8e schematically illustrate sector definitions for close contact analysis;

Fig. 9 illustrates energy differences between the tailor-made force field and the hybrid method;

Fig. 10 shows various conformations of cyclohexane-1,4-dione, wherein the gas phase conformations 1a and 1b as well as 2a and 2b are symmetry related;

Fig. 11 shows frames from the wide amplitude data set for the transition 2a->2b, wherein all molecules are contained in a simulation box with periodic boundary conditions which is not shown;

Fig. 12 presents lattice energies found for cyclohexane-1.4-dione with the tailor-made force field and the hybrid method; and

Fig. 13 shows a superposition of the experimental crystal structure of cyclohexane-1,4-dione and the corresponding structure after optimization with the hybrid method.

## 2.1 Force field atom types

In transferable force fields, force field atom types (FFATs) are used to limit the number of distinct interactions for which force field parameters have to be specified. Chemically similar atoms, for instance all atoms of the same element type and in the same hybridization state, are attributed the same FFATs, either manually or automatically using well-defined typing rules. The energy terms of a force field are defined with respect to the FFATs, i.e. for each type of interaction the force field does not only specify parameters and a mathematical function but also the FFATs of the atoms that participate in the interaction. The Dreiding force field, for example, distinguishes only 4 force field types for carbon atoms, namely C_1, C_2, C_R and C_3. Consequently, there is a maximum of 10 different bond stretch terms for carbon-carbon bonds and in practice even less since several bonds are described by the same parameters.

Our tailor-made force field is meant to describe the geometry and the energetics of a given molecule as closely as possible and the limitation to a small number of FFATs and energy terms is undesirable. However, it would be counterproductive to attribute an individual FFAT to every atom, i.e. to discard the concept of force field atom types altogether. The energy terms of a force field must be defined such that the same total energy is claculated for equivalent molecular conformations, which can be achieved by attributing the same FFATs to equivalent atoms. For a molecule with a methyl group, for example, the total energy should be the same for the three conformations that can be obtained by rotating the methyl group through 120˚. This constraint can be easily met by using the same FFATs for the three hydrogen atoms of the methyl group.

Unlike the FFATs of a transferable force field that are defined once and for all, the FFATs used with our tailor-made force field according to the invention are generated for every molecule independently. Since the choice of appropriate FFATs conditions all subsequent work with a tailor-made force field, we have automized the generation of FFATs on a molecule by molecule basis. The applied rules, in particular with respect to the issue of atomic equivalence, shall now be discussed.

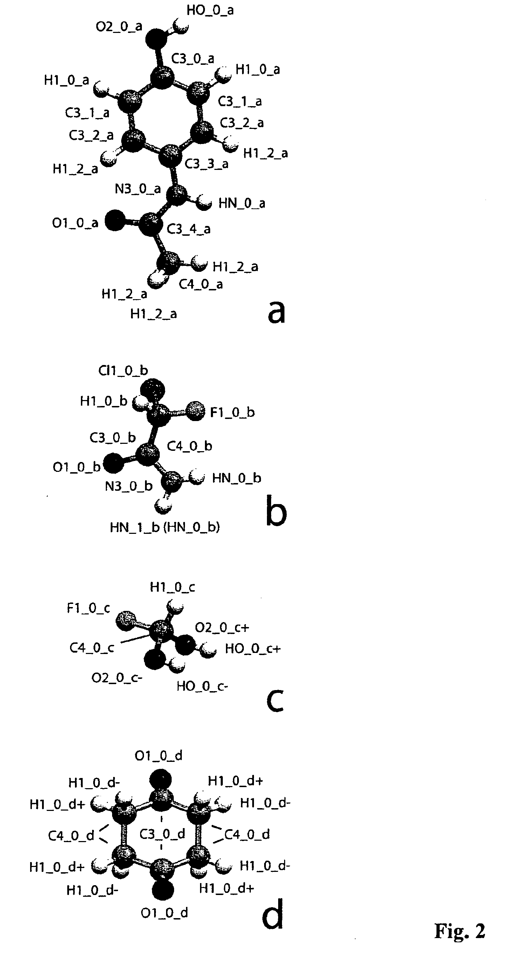

The names of most of our FFATs follow the scheme Xn_m_Ns, where X, n, m, N and s are the element symbol, the number of covalently bonded neighbours, a running number, a user-defined name for all FFATs belonging to the same molecule and an inversion indicator (see below), respectively. For hydrogen atoms involved in hydrogen bonding, we use a slightly different scheme of the type XY_m_Ns, where Y is the element symbol of the hydrogen bond donor. Both naming schemes differ only with respect to the primary FFAT, which we define as the beginning of the type name up to the first underscore. Some typical type names are shown in Fig. 2.

Two atoms, i and j, are considered to be equivalent, and thus attributed the same force field type, if there is an energetically possible atomic rearrangement that brings every atom onto a position formerly occupied by an atom of the same element type, with atom i ending up at the former location of atom j. The notion of 'energetically possible rearrangment' is related to the energy barriers that can be overcome in the molecular mechanics or dynamics calculations for which the FFATs are generated. If all atoms were allowed to move around freely, including bond breaking, all atoms of the same element type would have to be attributed the same FFAT according the above definition. In the energy range relevant for the structure prediction of molecular crystals, bond breaking and bond making can be neglected and the handedness of the atomic arrangement around a central atom with four covalently bonded neighbours does not change. In addition, rotations around $sp_2$-$sp_2$ double bonds are not possible in many cases. For computational applications, it is more convenient to transform the above statements to a set of conditions that can be checked against a given conformation of a molecule.

**Definition 1 (atomic equivalence):** Two atoms in a molecule, i and j, are considered to be equivalent, if a permutation P of all atoms exists that meets the following conditions:

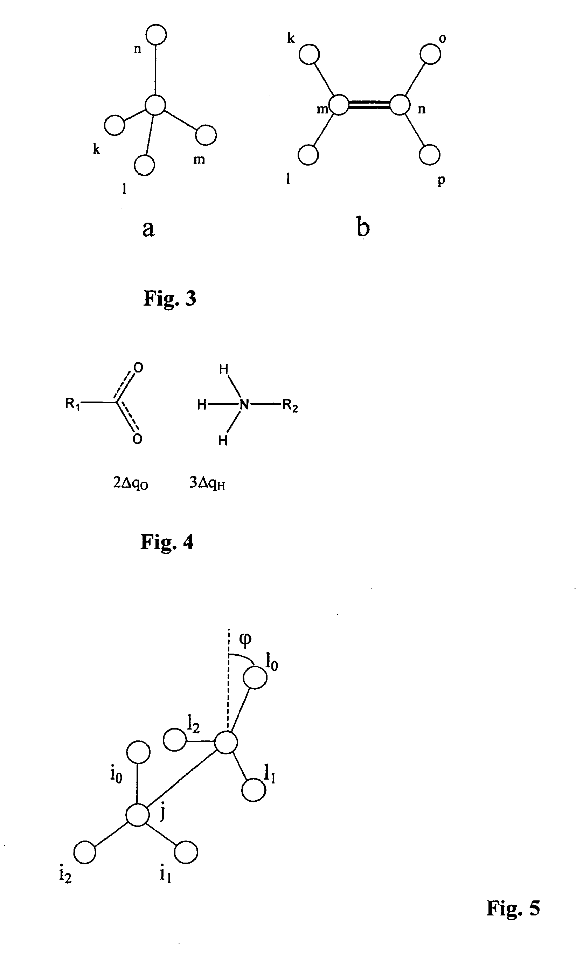

    a) **Atomic match:** i=P(j).
    b) **Element equivalence**: The atoms k and P(k) belong to the same element for all k.
    c) **Bond topology:** If two atoms, k and 1, are covalently bonded, so are the atoms P(k) and P(1), and vice versa.
    d) **Handedness**: For atoms with four covalently bonded neighbours, the permutation does not change the handedness (see Fig. 3a for atom numbering).

$$sign\left(\left(\vec{r}_n - \vec{r}_k\right) \cdot \left[\left(\vec{r}_m - \vec{r}_k\right) \times \left(\vec{r}_l - \vec{r}_k\right)\right]\right) = sign\left(\left(\vec{r}_{P(n)} - \vec{r}_{P(k)}\right) \cdot \left[\left(\vec{r}_{P(m)} - \vec{r}_{P(k)}\right) \times \left(\vec{r}_{P(l)} - \vec{r}_{P(k)}\right)\right]\right) \qquad (1)$$

In the above equation, $\vec{r}_k$ is the position of atom k in Cartesian coordinates.
    e) **$sp_2$-$sp_2$ rotations (optional):** Rotations around $sp_2$-$sp_2$ rotations are not allowed (see Fig. 3b for atom indices).

$$sign\left(\left[\left(\vec{r}_k - \vec{r}_m\right) \times \left(\vec{r}_l - \vec{r}_m\right)\right] \cdot \left[\left(\vec{r}_p - \vec{r}_n\right) \times \left(\vec{r}_o - \vec{r}_n\right)\right]\right)$$
$$= sign\left(\left[\left(\vec{r}_{P(k)} - \vec{r}_{P(m)}\right) \times \left(\vec{r}_{P(l)} - \vec{r}_{P(m)}\right)\right] \cdot \left[\left(\vec{r}_{P(p)} - \vec{r}_{P(n)}\right) \times \left(\vec{r}_{P(o)} - \vec{r}_{P(n)}\right)\right]\right) \qquad (2)$$

Fig. 2a shows the FFATs obtained for paracetamol according to the above rules. Fig. 2b presents the FFATs generated for another molecule with and without (FFAT in brackets) the optional condition e.

The concept of atom equivalence elaborated above requires one further extension to be satisfactory in all cases, as illustrated by Fig. 2c. The atoms O2_0_c+ and HO_0_c+ are not equivalent to the atoms O2_0_c- and HO_0_c-, because the permutation that exchanges these atoms does not comply to condition d of definition 1. However, it is intuitively clear that there has to be a symmetry relationship between the energy terms for the two OH groups which should be reflected by the choice of appropriate atom types. The two oxygen atoms and the two hydrogen atoms in Fig. 2c are inversion equivalent, whereby we call two atoms inversion equivalent if there is an inversion followed by an energetically possible rearrangement that brings every atom onto a position formerly occupied by an atom of the same element, with atom i ending up on the former position of atom j. In mathematical terms, we define inversion equivalence as follows:

    **Definition 2 (atomic inversion equivalence)**: Two atoms, i and j, are considered to be inversion equivalent, if definition 1 is applicable with the exception of condition d, which now takes a slightly different form:

d') **Handedness**: For atoms with for covalently bonded neighbours, the permutation changes the handedness (see Fig. 3a for atom indices)

$$sign\left((\vec{r}_n - \vec{r}_k) \cdot \left[(\vec{r}_m - \vec{r}_k) \times (\vec{r}_l - \vec{r}_k)\right]\right) = -sign\left((\vec{r}_{P(n)} - \vec{r}_{P(k)}) \cdot \left[(\vec{r}_{P(m)} - \vec{r}_{P(k)}) \times (\vec{r}_{P(l)} - \vec{r}_{P(k)})\right]\right) \quad (1')$$

If a molecular conformation can be mapped onto itself by a combination of an inversion and an energetically possible atomic rearrangement, force field calculations should yield the same total energy for the two atomic arrangements. One may be tempted to insure the expected result by attributing the same FFATs to inversion equivalent atoms, but this approach only works if the potential energy functions for torsions and inversions do not contain any uneven terms, because torsion angles and inversion distances change sign upon inversion (see Sections 2.7 and 2.8). To allow for uneven terms, we have made a different choice.

If two atoms are inversion equivalent but not equivalent, they are attributed FFATs that differ only at the last character, the inversion indicator s (see above) being set to '+' for one atom and '-' for the other. If an atom does not have a counterpart that is inversion equivalent without being equivalent, the inversion indicator is just an empty string. With respect to the definition of force field energy terms, the inversion indicator is used as follows. If a bond stretch term is defined for two covalently bonded atoms with FFATs $t_1$ and $t_2$, the same bond stretch terms is also applied to two covalently bonded atoms with FFATs $t_1^{-1}$ and $t_2^{-1}$, where the exponent indicates an operation that inverts the inversion indicator if it is not an empty string. Similarly, angle bend terms defined for three FFATs $t_1$, $t_2$ and $t_3$ are also valid for FFATs $t_1^{-1}$, $t_2^{-1}$ and $t_3^{-1}$. For torsion terms, the situation is slightly different. If a torsion term is defined for FFATs $t_1$, $t_2$, $t_3$ and $t_4$, there also is an equivalent energy terms for the FFATs $t_1^{-1}$, $t_2^{-1}$, $t_3^{-1}$ and $t_4^{-1}$, but the torsion angle is measure clockwise in the first case and counterclockwise in the second case. Inversion terms are dealt with in the same way as torsion terms, i.e. the measurement direction is inverted for the inversion equivalent FFATs. Fig. 2d shows the FFATs obtained for cyclohexane-1,4-dione using the concepts of atomic equivalence and inversion equivalence.

The automatic generation of a typing rule is at least as important as the automatic generation of FFATs. Typing rules can be used in computer programs to automatically attribute FFATs to every atom in a crystal structure. With our tailor-made force field, we use molecular typing rules that are applied to groups of covalently bonded atoms. A crystal structure is first decomposed into molecules, i.e. sets of atoms that contain all covalently bonded neighbours for each atom in the set. The algorithm then tries to number all atoms in each set in such a way that they meet one of the molecular typing rules of the tailor-made force field. A typical typing rule is presented in Table 1 for the molecule shown in Fig. 2b, whereby the optional condition e has been applied. For every atom in a molecule, the typing rule specifies an atom index, i, the force field type, $t_i$, the element, $e_i$, a conformation indicator, $\sigma_i$, and the indices of all covalently bonded neighbours, $b_{i,j}$. The conformation indicator can only take the values -1, 0 and 1. For a typing rule to apply to a set of atoms, the atoms must be numbered such that each atom has the element type and the bond partners specified by the typing rule. For atoms with 3 or 4 covalent bond partners, additional criteria apply if the conformation indicator is not zero. An atom, i, with 4 bond partners must meet the handedness criterion:

$$\sigma_i (\vec{r}_{b_{i,4}} - \vec{r}_{b_{i,1}}) \cdot \left[(\vec{r}_{b_{i,3}} - \vec{r}_{b_{i,1}}) \times (\vec{r}_{b_{i,2}} - \vec{r}_{b_{i,1}})\right] > 0 \quad (3)$$

Two covalently bonded atoms, i and j, with three bond partners each and non-zero conformation indicators must fulfil the $sp_2$-$sp_2$ rotation criterion.

$$\sigma_i \sigma_j \left[(\vec{r}_{b_{i,2}} - \vec{r}_{b_{i,1}}) \times (\vec{r}_{b_{i,3}} - \vec{r}_{b_{i,1}})\right] \cdot \left[(\vec{r}_{b_{j,2}} - \vec{r}_{b_{j,1}}) \times (\vec{r}_{b_{j,3}} - \vec{r}_{b_{j,1}})\right] > 0 \quad (4)$$

We close the subsection with some remarks about chiral molecules. A force field should yield the same energy for a chiral molecule and its mirror copy, and a single molecular typing rule should cover both cases. Therefore, if a group of atoms cannot be types with the 'straight' typing rule, we also apply the 'inverse' typing rule, in which the signs of all conformation indicators are inverted. We again have to take into account that torsion angles and inversion distances change sign upon inversion. Hence, for a chiral molecule typed with the 'straight' typing rule and its mirror copy typed with the 'inverse' typing rule, torsion angles and inversion distances must be measured in opposite directions.

**TABLE 1:** Molecular typing rule for the molecule shown in Fig. 2b (see text for details).

| i | $t_i$ | $e_i$ | $\sigma_i$ | $b_{i,j}$ |
|---|---|---|---|---|
| 1 | H1_0_b | H | 0 | 4 |
| 2 | C1_0_b | Cl | 0 | 4 |
| 3 | F1_0_b | F | 0 | 4 |
| 4 | C4_0_b | C | -1 | 5 3 2 1 |
| 5 | C3_0_b | C | 1 | 746 |
| 6 | O1_0_b | O | 0 | 5 |
| 7 | N3_0_b | N | -1 | 589 |
| 8 | HN_0_b | H | 0 | 7 |
| 9 | HN_1_b | N | 0 | 7 |

### 2.2 Electrostatic interactions

The electrostatic interaction between two atoms, i and j, is described in terms of a Coulomb potential for fixed atomic point charges:

$$E_{ij} = C \frac{Q_i Q_j}{R_{ij}} \text{ with C=322.0637 kcal/mol.} \tag{5}$$

Electrostatic interactions between next and second next covalently bonded neighbours are not taken into account, and the total electrostatic energy of a crystal is computed by means of Ewald summation with automatic parameter estimation.

The handling of the atomic point charges merits some further comments. The atomic charges used with many force fields are not related to force field parameters, but determined by other methods and stored as properties of the structural model. This choice was unavoidable for the early force fields, where the number of FFATs is too small to allow for the accurate enough description of the molecular charge distribution. Using the procedure for the generation of FFATs outlined in the previous subsection, atomic charges can be treated as force field parameters without loss of accuracy, and we have chosen this option because it facilitates the force field usage and refinement.

In our tailor-made force field, atomic charges are defined via bond increments.[18] For every pair of covalently bonded atoms, i and j, with FFATS $t_i \neq t_j$, a bond increment $\delta_{t_i t_j}$ must be defined which contributes the amounts $+\delta$ and $-\delta$ to the total charge of atoms i and j, respectively. For neutral molecules, bond increments offer the same flexibility for the description of the molecular charge distribution as charges directly associated with FFATs, but present the additional advantage that the charge neutrality constraint is obeyed by definition.

Even though our validation work has so far focused on neutral molecules, we have generalized the concept of bond increments by the introduction of charge transfer increments in order to make it applicable to molecular salts. Consider the situation depicted in Fig. 4. The left molecule donates a proton to the right molecule and acquires a negative net charge. This charge is actually less than the formal charge of -1.0, because the positively charged $NH_3$ group captures some of the electronic charge density around the negatively charged $CO_2$ group. The charge transfer between the two groups can be described by defining a charge transfer increment for the oxygen and hydrogen atoms.

A charge transfer increment $\tilde{\delta}_{t_1 t_2}$ can be defined for any two FFATs, t1 and t2, whereby the corresponding atoms to not have to be bonded. The charges transfer increment transfers the charge $\Delta q_{t_1}$ and $\Delta q_{t_2}$ to every atom with the FFAT $t_1$ and $t_2$, respectively, with:

$$\Delta q_{t_1} = +\frac{N_{t_1} + N_{t_2}}{2N_{t_1}} \tilde{\delta}_{t_1 t_2} \qquad (6)$$

and

$$\Delta q_{t_2} = -\frac{N_{t_1} + N_{t_2}}{2N_{t_2}} \tilde{\delta}_{t_1 t_2} . \qquad (7)$$

Here $N_{t1}$ and $N_{t2}$ are the number of atoms with the corresponding FFAT in the unit cell. The use of eqs. 6 and 7 insures that the total charge of the unit cell remains zero regardless of the stoechiometry and the amount of charge transfer.

If a bond increment or a charge transfer increment is defined for two FFATs , $t_1$ and $t_2$, it is also valid for the inverse equivalent FFATs, $t_1^{-1}$ and $t_2^{-1}$.

**2.3 Van der Waals interactions**

To describe long range dispersive interactions and hard core repulsions we use isotropic atom-atom pair potentials of the exponential-6 type:

$$E_{ij} = A_{ij} \exp(-B_{ij} R_{ij}) + C_{6,ij} \frac{1}{R_{ij}^6} \qquad (8)$$

The summation over pair potentials is carried out in direct space and interactions beyond the direct space cut off are taken into account by means of a continuum correction. The $1/R^6$ term is damped at short interatomic distances to avoid divergence.

The parameters A, B and C must be defined explicitly for all homo-atomic interactions. Parameters for hetero-atomic interactions are either specified explicitly or derived from the homo-atomic parameters by means of combination rules:

$$A_{ij} = \sqrt{A_{ii} A_{jj}} \qquad (9)$$

$$B_{ij} = \frac{1}{2}(B_{ii} + B_{jj}) \qquad (10)$$

$$C_{6,i,j} = \frac{2(C_{6,i,i}^2 C_{6,j,j}^2 N_{eff,i} N_{eff,j})^{1/3}}{(C_{6,i,i} N_{eff,j}^2)^{1/3} + (C_{6,j,j} N_{eff,i}^2)^{1/3}} \qquad (11)$$

In eq. 11, $N_{eff}$ refers to the effective electron numbers according to Halgren[11].

In general, we use the same VdW parameters A and B for all atoms of the same element, with exception of hydrogen atoms involved in hydrogen bonding, which are dealt with separately. Our $C_6$ parameters depend on the primary FFATs. We typically take the starting values for the $C_6$ parameters from the hybrid method and use a single scale factor for all of them in the parameter refinement. Tests for several molecules have shown that the use of individual vdW parameters for every FFAT does not increase the overall accuracy of the tailor-made force field significantly. The explicit refinement of certain hetero-atomic interaction parameters, on the other hand, strongly improves the overall accuracy. Hetero-atomic interaction parameters should be refined at least for all atom pairs involving hydrogen atoms.

### 2.4 Hydrogen bonding terms

Our tailor made force field allows for the definition of hydrogen bond terms using the same potential energy function as the Dreiding force field:

$$E_{hb} = D_{hb} \left[ 5 \left( \frac{R_{hb}}{R_{DA}} \right)^{12} - 6 \left( \frac{R_{hb}}{R_{DA}} \right)^{10} \right] \cos^4 (\theta_{DHA}) . \qquad (12)$$

Here $\theta_{DHA}$ is the bond angle between the hydrogen donor (D), the hydrogen (H), and the hydrogen acceptor (A), while $R_{DA}$ is the distance between the donor and acceptor atoms in Å. $D_{hb}$ is the depths of the hydrogen bond potential and $R_{hb}$ is the equilibrium distance.

After several parameter refinements, we have come to the conclusion that special hydrogen bond parameters are not required, if bond increments and vdW parameters, including hetero-atomic parameters for the hydrogen (H) - acceptor (A) interaction, are optimized simultaneously.

### 2.5 Bond stretch terms

Harmonic bond stretch terms are evaluated for all pairs of covalently bonded atoms, i and j, with FFATs, $t_i$ and $t_j$:

$$E_{ij} = \frac{1}{2} K_{t_i t_j} (R_{ij} - R_{t_i t_j,0})^2 \qquad (13)$$

Starting values for the parameters $K_{t_i t_j}$ and $R_{t_i t_j,0}$ are taken from the Dreiding force field.

### 2.6 Angle bend terms

Harmonic angle bend terms are evaluated for all pairs of atoms, i and k, covalently bonded to a central atom, j :

$$E_{ijk} = \frac{1}{2} K_{t_i t_j t_k} (\theta_{ijk} - \theta_{t_i t_j t_k,0})^2 \qquad (14)$$

Here $\theta_{ijk}$ is the angle between the bonds i-j and j-k. Starting values for the parameters $K_{t_i t_j}$ and $\theta_{t_i t_j t_k,0}$ are taken from the Dreiding force field.

### 2.7 Torsion terms

In most transferable force fields, torsion terms are evaluated for all quadriplets of atoms i, j, k and 1 connected via three adjacent covalent bonds, whereby each torsion term is an even function of the corresponding torsion angle, for instance:

$$E_{ijkl} = \frac{1}{2} V_{t_i t_j t_k t_l} \left[ 1 - S_{t_i t_j t_k t_l} \cos \left( p_{t_i t_j t_k t_l} \varphi_{ijkl} \right) \right] \qquad (15)$$

Here, V is the torsion barrier, S can take the values +1 and -1 and p characterizes the periodicity of the torsion potential. The torsion angle $\varphi_{ijkl}$ is measured clockwise when looking down the j-k bond (see Fig. 5). Since there are several different choices for the terminal atoms, the overall torsion potential for the rotation around a central bond results in general from the superposition of several different torsion terms and may have an uneven component. The limitation to even torsion terms offers the advantage that the sign change of the torsion angles upon inversion of the atomic coordinates must not be worried about.

The presence of several torsion terms for every central bond is not a problem if the corresponding force field parameters result from an educated guess or are fitted to a large body of data involving only a small number of FFATs. In the case of our tailor-made force field with its large number of FFATs, on the contrary, the number of independent torsion terms needs to be reduced to avoid a high level of redundancy, which would complicate the parameter refinement significantly.

We have therefore adopted a different strategy, where the torsion around a central bond is characterized by a single, overall torsion angle which depends on the coordinates of the two central atoms and all their covalently bonded neighbours. One of the individual torsion angles is chosen as the 'representative' torsion angle and all other individual torsion angles are 'projected' onto the representative torsion angle. The overall torsion angle is the average over all projected torsion angles.

$$\varphi_{overall} = \frac{1}{AB}\sum_{a=0}^{A-1}\sum_{b=0}^{B-1}\left[P_{]-180,180]}\left(\varphi_{i_a jkl_b} + \frac{360°}{A}a - \frac{360°}{B}b - \varphi_{i_0 jkl_0}\right) + \varphi_{i_0 jkl_0}\right] \qquad (16)$$

In the above equation, $P_{]-180,180]}$ is a projection operator that adds a multiple of 360˚ to its argument such that the result falls into the interval ]-180˚,180˚]. A and B are the external number of covalently bonded neighbours of the atoms j and k, respectively (see Fig. 5). The torsion energy as a function of the overall torsion angle is defined in terms of a Fourier series:

$$E_{overall,tor} = E_0 + \sum_{n=1} c_n \cos[pn(\varphi_{overall} - \varphi_n)] \qquad (17)$$

For the sake of simplicity, we have omitted the indices which indicate the dependence of the parameters $E_0$, $c_n$, p and $\varphi_n$ on the FFATs of the involved atoms. Since there now is only a single torsion term for each central bond, the uneven component of the overall torsion potential must be included explicitly in the overall torsion term. Hence, the angles $\varphi_n$ are in general different from 0˚ or 180˚. The uneven component requires a special treatment of torsion terms related by inversion symmetry (see section 2.1). Depending on the molecular symmetry, as reflected by the FFATs of the tailor-made force field, the angles $\varphi_n$ may have to be equal to 0˚ or 180˚. This is the case when the FFATs of the external atoms obey the equalities $t_{i_a} = t_{i_{A-o}}$ and $t_{i_b} = t_{i_{B-b}}$. The periodicity p in eq. 17 depends on the FFATs of the atoms $i_a$, ..., $i_{A-1}$ and $l_b$,...$l_{B-1}$. Let $p_i$ and $p_l$ be the maximum number of identical subsequences that the sequences of FFATs $t_{i_0}$,...., $t_{i_{A-1}}$ and $t_{l_0}$,...., $t_{l_{B-1}}$ can be decomposed into. Then p is the smallest common multiple of $p_i$ and $p_l$.

## 2.8 Inversion terms

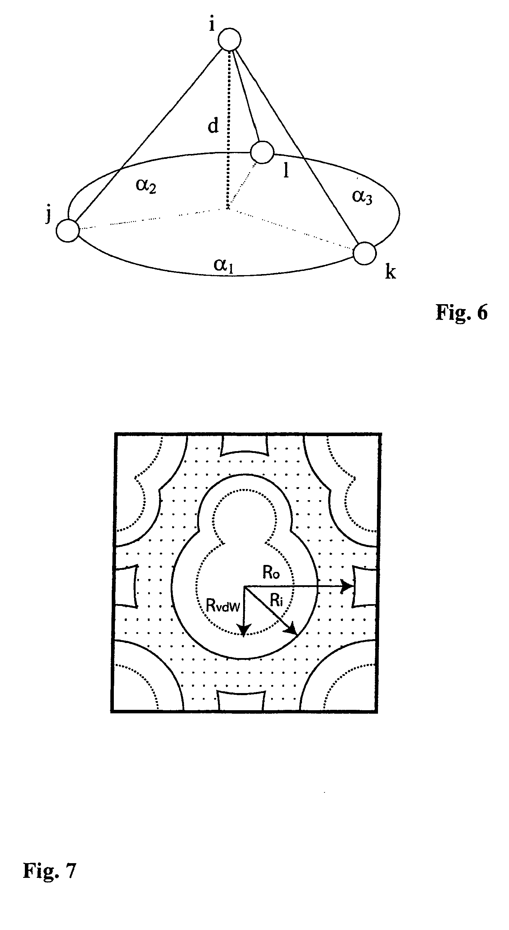

In transferable force field, inversion terms typically are an even function of a parameter that measures the out of plane motion of the central atom surrounded by three covalently bonded neighbours (see Fig 6). The Dreiding force field, for instances, uses inversion terms where $\psi_{ijkl}$ is the angle between the i-j bond and the j-k-l plane. The limitation to even inversion terms is not always appropriate. In particular in larger molecules, the central atom may systematically prefer one particular side of the plane through its neighbours because of steric effects. Such a situation is difficult to parameterize if uneven terms are not allowed. Inversion terms such as the one shown in eq. 18 have the additional disadvantage that the mathematical description of the inversion potential is only symmetric with respect to the three surrounding atoms if three individual inversion terms are taken into account for each central atom, a redundancy that needs to be avoided in our tailor-made force field.

We parameterize inversion potentials in terms of a Taylor series of the center-to-plane distance (inversion distance) d, which in invariant with respect to a cyclic permutation of the surrounding atoms:

$$d_{ijkl} = \left(\vec{r}_l - \vec{r}_i\right) \cdot \frac{\left(\vec{r}_k - \vec{r}_i\right) \times \left(\vec{r}_j - \vec{r}_i\right)}{\left|\left(\vec{r}_k - \vec{r}_i\right) \times \left(\vec{r}_j - \vec{r}_i\right)\right|} \tag{19}$$

$$E_{overall,inv} = \sum_{n=0} c_{t_i t_j t_k t_l, n} d_{ijkl}{}^n \tag{20}$$

For every central atom, there is only a single overall inversion term. The sign of the inversion distance is only well defined if the order of the surrounding atoms is unambiguous, i.e. if the three neighbours have different FFATs. If this is not the case, uneven terms are not allowed in eq. 20.

### 2.9 Angle bend - inversion coupling

We have observed that the conformational flexibility around atoms with three covalently bonded neighbours is not always described sufficiently well by a combination of separate inversion and angle bend terms. We have therefore introduced a combined angle bend - inversion term which allows for a more detailed description of the potential energy:

$$E_{term} = E_{inv}(d) + \frac{1}{2} k_1(d)(\alpha_1 - \alpha_{1,eq}(d))^2 + \frac{1}{2} k_2(d)(\alpha_2 - \alpha_{2,eq}(d))^2 + \frac{1}{2} k_3(d)(\alpha_3 - \alpha_{3,eq}(d))^2$$

$$\tag{21}$$

$$\alpha_{3,eq}(d) = 360° - \alpha_{1,eq}(d) - \alpha_{2,eq}(d) \tag{22}$$

The terms $E_{inv}(d)$, $k_1(d)$, $k_2(d)$, $k_3(d)$, $\alpha_{1,eq}(d)$ and $\alpha_{2,eq}(d)$ are defined as Taylor series of the inversion distance d in analogy to eq. 20. The angles $\alpha_1$, $\alpha_2$ and $\alpha_3$ are obtained by projection of the bonds i-j, i-k and i-l onto the plane trough the surrounding atoms (see Fig. 6). If the FFATs of two or more surrounding atoms are identical, symmetry constraints apply to $k_1$, $k_2$, $k_3$, $\alpha_{1,eq}$ and $\alpha_{2,eq}$.

### 2.10 Intramolecular Coulomb and vdW scale factors

In common force fields, intramolecular Coulomb and vdW interactions between first and second next neighbours are not taken into account. From third next neighbours onwards, intramolecular interactions are evaluated in the same way as intermolecular interactions, whereby all third next neighbour interactions may sometimes be scaled down by a single scale factor. Our own force field fitting work indicates that the full consideration of Coulomb and vdW interactions from third next neighbours onwards is a valid choice for most atoms in a molecule, but there can be exceptions. For example, we had to deal with the case of a bulky side group attached to an aromatic ring, where the contribution of the intramolecular Coulomb and vdW interactions to the force constant of the in-plane bend mode was bigger than the target force constant according to the reference data, thus giving rise to negative force constants for some of the angle bend terms after parameter refinement. To avoid 'unphysical' force field parameters in cases like this, we have implemented a general approach for the scaling of intramolecular Coulomb and vdW interactions on a case by case basis.

When implementing the method according to the invention in a computer program, it is advantageous to allow for the definition of one or more sequences of FFATs, whereby each sequence is accompanied by two scale factors for the Coulomb and the vdW interactions. For every chain of covalently bonded atoms in agreement with one of the sequences of FFATs, the corresponding scale factors are applied to the interactions between the terminal atoms of the chain. Each FFAT in a sequence may contain wildcards, namely 'X' for any FFAT, '*' for any character string or '?' for any character. The scaling is not necessarily limited to third next neighbour interactions and may also be used to obtain a more accurate description of intramolecular hydrogen bonds in cases where the hydrogen and the acceptor are many covalent bonds apart.

### 3. Reference data

An extensive set of reference data is required to determine appropriate values for the many force field parameters described in the previous section. Our hybrid method[4], which combines DFT calculations by means of the VASP program with an empirical van der Waals correction, is the natural choice for the generation of reference data, because up to now it is the only approach that has proven accurate enough for the lattice energy ranking of molecular crystals. VASP uses a plane wave basis set and periodic boundary conditions. For the refinement of some force field parameters, in particular those of intramolecular energy terms, it would in principle have been more convenient to use a DFT code based on atomic orbitals and infinite boundary conditions. However, this option has been discarded, because it increases the number of third party components and, more importantly, because it involves the non-trivial task of deriving an empirical van der Waals correction for an isolated molecule DFT code.

The hybrid method produces three basic data types: the electrostatic potential at certain points in the unit cell, the lattice energy and the first derivatives of the lattice energy with respect to the unit cell parameters and the atomic coordinates. Bases on these basic data types, it is possible to construct various data sets which are sensitive to different force field parameters and intervene at different stages of the parameter refinement process. We shall now briefly discuss the data sets currently in use.

**Electrostatic data**: The electrostatic potential is evaluated for a set of points in a unit cell. The points are taken from a regular grid with an approximate spacing $\Delta$, from which all points have been removed that are closer than $r_{vdw}*f_{inner}$ to any atomic nucleus and further away than $r_{vdw}*f_{outer}$ from all atomic nuclei (see Fig. 7). Symmetry related grid points are represented by a single grid point with the appropriate multiplicity. We typically use the settings $\Delta=0.2$ Å, $f_{inner}=1.5$ and $f_{outer}=2.5$. A factor of 1.5 for the inner radius is required, because the electrostatic potential must be evaluated outside the molecular electron cloud. In a densely packed crystal structure, there is hardly ever any empty space 1.5 vdW radii away from the atomic positions. We therefore use crystal structures that have been expanded such that the distance between atoms on neighbouring molecules obeys the relationship

$$ r_{ij} = (r_{vdW,i} + r_{vdW,i})(1 + f_{exp\,ansion}) . \qquad (23) $$

During the expansion, the molecular geometries and the fractional coordinates of the molecular positions in the unit cell are held constant. The three factors $f_{inner}$, $f_{outer}$ and $f_{expansion}$ must be chosen consistently, and we typically set $f_{expansion}$ such that the shell of acceptable points around a molecule does not significantly overlap with the exclusion zone of a neighbouring molecule, which leads to the inequality

$$ r_{vdW,i} * f_{outer} + r_{vdW,j} * f_{inner} \leq (r_{vdW,i} + r_{vdW,j})(1 + f_{exp\,ansion}) \qquad (24) $$

for all atoms i and j. With values for $f_{inner}$ and $f_{outer}$ as mentioned above, $f_{expansion}=1$ is a reasonable choice if all atoms have similar vdW radii, while $f_{expansion}=1.5$ should be used if the vdW radii are very dissimilar.

**Minimization data**: Rigid body minimization data are used for the parameterization of non-bonded interactions. In addition, minimization data make it possible to feed the results of an energy ranking study with the hybrid method back into the parameter refinement. A lattice energy minimization is carried out for every member of a set of crystal structures. All crystal structures have to consist of the same molecules, such that their lattice energies are comparable. Throughout the remainder of this work we will call such a set a polymorph set. Minimization data, like other data types described below, typically consist of several polymorph sets. The lattice energy minimizations may be carried out for rigid or flexible molecules and under different pressures, but the conditions must be the same for every structure of the polymorph set. The tailor made force field should reproduce the lattice energy differences between the optimized crystal structures and yield vanishing forces, since every structures corresponds to a potential energy minimum. As we will see in section 4, the integration of the force criterion into the deviation function requires a second derivatives matrix (Hessian matrix). A starting guess of the Hessian matrix is calculated numerically for all crystal structures using a force field. For the structure optimization with the hybrid method we use a quasi-Newton algorithm which gradually improves the Hessian matrix.

**Packing data**: Packing data are used for the refinement of non-bonded interactions. They are similar to minimization data, but offer some additional features. Molecules are always treated as rigid bodies, and the molecular geometry must be the same for all structures of the polymorph set. After each lattice energy minimization, the lattice energy and its first derivatives are evaluated for a set of points around the local minimum. For each degree of freedom (lattice deformations, whole molecule translations and rotations), two points are considered corresponding to the positive and the negative displacement direction. The distance with respect to the local minimum is chosen such that the energy increases by roughly

$$\Delta E = \frac{1}{2} kT_{RT} . \qquad (25)$$

Hence, the potential energy well is probed on a length scale which corresponds to the thermal occupation of the configuration space. Quantum effects to not need to be considered, because the energy spacing of the vibrational modes involving whole molecule translations and rotations is sufficiently small. For the exploitation of packing data in the deviation function, a Hessian matrix is required which is calculated numerically from the energy derivatives at the points around the potential energy minimum.

**Expansion data**: Expansion data are useful for the calibration of the depth of non-bonded potentials. For a set of crystal structures, every member is expanded by a certain amount, whereby the molecular geometry and the molecular position in fractional coordinates are held constant. The lattice energy is evaluated for the original structure, the final structure and eventually some intermediate crystal packings.

**Conformation data**: Conformation data are used to derive intramolecular parameters that accurately describe the principal conformations of a given molecule, including the potential energy well around the local minima. Conformation data are generated for a polymorph set, whereby every member corresponds to one particular conformation of a molecule in a P1 unit cell. The size of the unit cell is chosen such that there is some empty space around the molecule. In a first step, the molecular geometry is optimized, keeping the molecular position and orientation as well as the cell parameters constant. The minimization is carried out with a quasi-Newton algorithm and the second derivatives matrix resulting from the minimization is used for the calculation of approximate vibrational eigenmodes. For every eigenmode with an angular frequency $\omega$, the lattice energy and the first derivatives are calculated at two points away from the minimum, whereby the distance is chosen to correspond to an energy increase equal to the average potential energy of the eigenmode

$$\Delta E = h\omega \frac{0.5 + \exp(-\frac{-h\omega}{kT_{RT}})}{1 - \exp(-\frac{h\omega}{kT_{RT}})} . \qquad (26)$$

As for packing data, the potential energy well is probed in the region of the configuration space with is thermally occupied, but this time quantum effects including zero-point motions are explicitly taken into account. Again, a Hessian matrix is required for the exploitation of the conformation data which is calculated numerically from the forces around the minimum.

**Wide amplitude data**: Wide amplitude data characterize the energy barriers between molecular conformations. Each data set consists of a series of frames which contain a single molecule (in a large P1 unit cell) which gradually evolves from one conformation to another. Conformational changes are produced by adding additional constraint potentials to a force field or to the hybrid method. Constraint potentials are nothing else but normal energy terms with very high force constants. Constraint potentials can be defined for torsion angles, for inversion distances and for rings of covalently bonded atoms. In the last case, constraint potentials are defined for all ring torsions. To generate the various frames of a wide amplitude data set, the reference parameters of the constraint terms (target values for torsion angles or inversion distances) are changed stepwise from the values of one conformation to the values of another conformation. Each frame is first optimized with a force field, and a numerial second derivatives matrix is calculated. In a second step, the frame is optimized again with the hybrid method by means of a quasi-Newton algorithm which uses the Hessian matrix determined previously. For every frame, we store the lattice energy, the Hessian matrix and the constraint terms. The tailor-made force field must reproduce the energy differences between the different frames and yield vanishing energy derivatives for every frame if the constraint terms are taken into account. When a single degree of freedom is driven from one value to another, it may happen that another degree of freedom crosses an energy barrier and changes abruptly by a large amount, which leads to an energy discontinuity and complicates the interpretation of the wide amplitude data. We therefore typically define constraint terms for all relevant wide amplitude motions in a molecule. One of these constraint terms is driven to produce the wide amplitude date set, while the others are held constant to stabilize the rest of the molecule.

**Reverse minimization data**: This data type is used to remove false energy minima from the potential energy hypersurface of the tailor-made force field. After a first parameter refinement, it may happen that the tailor-made force field predicts energy minima for molecular conformations or packings which in reality are unstable. Such false minima may be spotted following calculations with the hybrid method or based on personal experience. To remove false minima, energies and gradients calculated with the hybrid method at the corresponding locations must be fed back into the parameter refinement. To generate reverse minimization data, we select some true and some false minima, optimize the corresponding structures with the tailor-made force field and calculate the Hessian matrix. In a second step, single point energy and gradient calculations with the hybrid method are carried out.

It is important to understand that the aforementioned data sets make it possible to split the force field parameters into several groups which are refined separately. The order of the parameter refinement is of curial importance. First, bond increments are derived from the electrostatic data. Then, the vdW parameters are fitted to rigid molecule minimization data, eventually complemented by packing data, and expansion data. Except for a constant energy offset per molecule which is treated as an adjustable parameter at this stage, the three data types do not depend on intramolecular energy terms because the molecule geometry is kept constant. In a third step, bond increments and vdW parameters are optimized together, using electrostatic data, minimization data, packing data and expansion data. In a forth step, the intramolecular energy terms are fitted to conformation data only, with the bond increments and vdW parameters being held constant. The conformation data generated with the hybrid method contain a contribution from the intermolecular interactions between the translational copies of each molecule. In the force field parameter refinement, this contribution is compensated by the Coulomb and vdW terms which have already been determined accurately at this stage. Since the empirical description of Coulomb and vdW parameters is not perfect, a small error is introduced in the intramolecular energy terms which is related to the uncompensated part of the intermolecular interactions. At this point, the tailor-made force field is already fairly accurate, but the potential energy barriers between molecular conformations are not yet well defined. The intermediate tailor-made force field is used for the force field calculations involved in the generation of wide amplitude data. This choice minimized the CPU time requirements of the hybrid method, since the pre-optimized frames and the corresponding numerical Hessians provide an excellent starting point. In a final refinement step, the intramolecular energy terms are fitted again, now taking into account conformation data and wide amplitude data.

The selection of rigid molecule packings for the generation of minimization data is a subtle issue which deserves a detailed discussion. We have already pointed out in section 2.3 that many hetero-atomic vdW parameter should be explicitly refined. As a consequence, the reference data and in particular the rigid molecule minimization data must be chosen such that all vdW potentials are well defined over the full range of intermolecular distances. Large interatomic separations are not a problem, because they occur in large numbers, but short contacts are 'rare events' and special care must be taken to insure complete coverage.

The necessity to cover all possible short contacts is further complicated by the fact that most organic molecules consist of a majority of carbon and hydrogen atoms, complemented by a small amount of other elements such as oxygen, nitrogen, sulphur, fluorine or and chlorine. To improve the coverage of close contacts between these minority atoms, it is advisable to construct several smaller molecules which contain an increased portion of minority atoms and exhibit the same chemical motives of the full molecule. For the sake of simplicity, we will call these molecules molecular fragments in all subsequent discussions. In the parameter refinement, the same vdW parameters are used for the molecular fragments and the full molecule. If appropriate, the molecular fragments and the full molecule may also share some bond increments. Electrostatic data have to be generated for all molecular fragments.

We currently generate crystal packings for all molecule fragments, all pairs of fragments and the full molecule if it is not too large. The structure generation is carried out with rigid molecules in all space groups with no more than two symmetry copies per reduced unit cell (P1, P-1, P2, P21, Pm, Pc, C2, Cm, Cc). The structure generation algorithm requires a force field for intermolecular interactions. We use a combination of bond increments fitted to electrostatic potential data and vdW parameters taken either from the Dreiding force field or from a previous force field parameter refinement for a related molecule. All generated crystal packings result from a rigid molecule optimization of the unit cell.

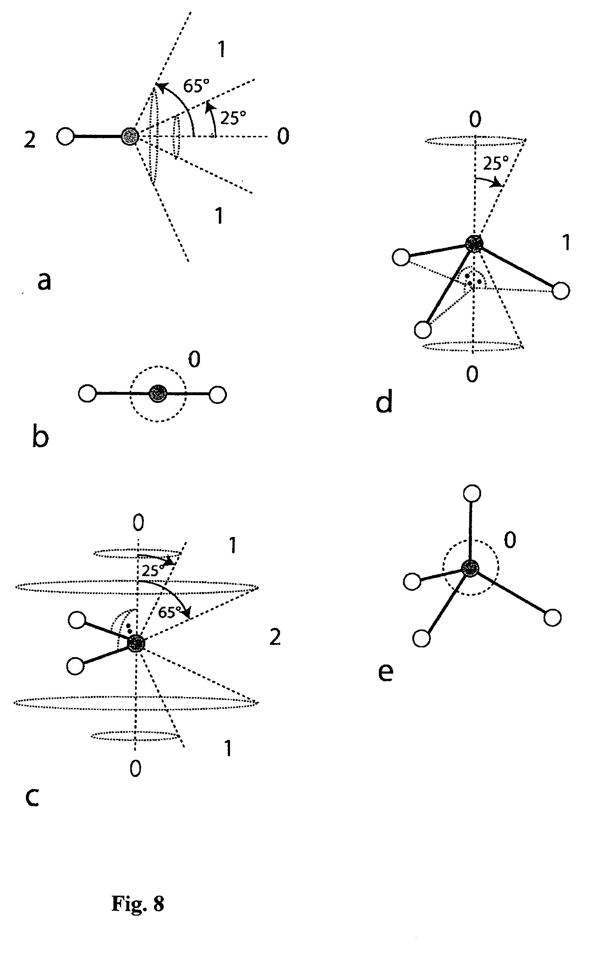

**Structure selection algorithm**: It would be far too time consuming to prepare minimization data for all generated crystal structures. Instead, it is necessary to select a set of structures that covers all possible close contacts while being relatively small. We have implemented an automatic selection procedure which roughly works as follows. Around every atom we define several sectors according to the number of covalently bonded neighbours (see Fig. 8). Fig. 8 distinguishes 5 different cases: atoms with one covalent bond (3 sectors), atoms with two collinear covalent bonds (1 sector), atoms with two non-collinear bonds (3 sectors), atoms with three covalent bonds (2 sectors), atoms with more than three covalent bonds (1 sector) The angular values shown in Fig. 8 have been added for the purpose of illustration only and different values may be used in an embodiment of the invention. All interatomic distances are divided into several catagories, whereby every category consists of the primary FFAT of a first atom, one of its sectors and the primary FFAT of a second atom. In a first step, the selection algorithm runs through all crystal structures and determines the shortest interatomic separation $r_{min,\zeta}$ for every distance category $\zeta$. In a second step, the algorithm runs through all crystal structures again and removes the current structure if for every distance category there are at least n remaining crystal structures with a close contact

$$r_\zeta \leq r_{min,\zeta} * (1+t) \tag{27}$$

We typically use n=3 et t=0.05. The result of the above procedure strongly depends on the order in which the various crystal structures are dealt with and we classify all crystal structures with respect to a risk factor, whereby structures with a high risk factor are removed first. The risk we are referring to is a large structural change which may occur when the selected structures are optimized with the hybrid method to produce rigid molecule minimization data. Each selected crystal structure is supposed to covers a certain number of close contacts, and a large structural change is likely to invalidate the very reason for which the structure was selected.

If the rigid molecule minimization data contain only crystal structures optimized under ambient pressure, the resulting vdW potentials are in general inappropriate for the description of intramolecular third next neighbour interactions. Indeed, intramolecular third next neighbour distances are significantly smaller than typical intermolecular distances under ambient pressure and an important portion of the distance range is not covered by the data set. To improve the description of third next neighbour interactions, we generate rigid molecule minimization data not only under ambient pressure but also at 5 GPa. At this pressure, the closest intermolecule distances are roughly equal to typical intramolecular third neighbour distances.

Rigid body minimization data and packing data can both be used for the refinement of non-bonded interactions. Packing data provide a more detailed characterization of each crystal packing, but also require significantly more time to be generated. If the number of distance categories to be considered is small, packing data are the preferred choice. In most cases, however, the number of distance categories is rather high and rigid body minimization data are the only practical option, because a large number of crystal structures is required to cover all relevant close contacts.

The need for the reference data to cover all relevant atomic configurations is not limited to minimization data, but applies to all data types. Electrostatic data and conformation data must be generated for all relevant conformations of a molecule. Wide amplitude data are required for all relevant conformational changes. At present, the responsibility for the generation of all required data sets lies with the user, but a conformer analysis tool has been implemented to facilitate the task.

## 4. Deviation function

For force field parameter refinement by means of a computer algorithm, it is advantageous to define a mathematical function that characterized the disagreements between the various reference data types and their counterparts calculated with the tailor-made force field by a single number. This deviation function D needs to be constructed in such a way that it is zero for a perfect match and larger than zero otherwise. The problem with the construction of the deviation function is that is has to combine a multitude of disparate data types (electrostatic potential, energies and forces at the potential energy minimum and at surrounding points, data for densely packed and expanded crystal structures, data for the full molecule and smaller fragments) in a balanced way such that no data type dominates the others.

In order to assess the contribution from the various data types on a common scale, we have made the choice to express all disagreements in terms of energy errors per atom. Whenever possible, the energy errors are calculated in such a way that there is a direct link with the lattice energy ranking of molecular crystals. The deviation function is the weighted average over several energy errors:

$$D(\vec{q}) = \frac{1}{W} \Big[\ w_{es} \frac{e_{es}^2(\vec{q})}{e_{ref}^2} + w_{m,1} \frac{e_{m,1}^2(\vec{q})}{e_{ref}^2} + w_{m,2} \frac{e_{m,2}(\vec{q})}{e_{ref}} + w_{p,1} \frac{e_{p,1}^2(\vec{q})}{e_{ref}^2}$$

$$+ w_{p,2} \frac{e_{p,2}(\vec{q})}{e_{ref}} + w_{p,3} \frac{e_{p,3}^2(\vec{q})}{e_{ref}^2} + w_{p,4} \frac{e_{p,4}(\vec{q})}{e_{ref}} + w_{e,1} \frac{e_{e,1}^2(\vec{q})}{e_{ref}^2} + w_{c,1} \frac{e_{c,1}^2(\vec{q})}{e_{ref}^2}$$

$$+ w_{c,2} \frac{e_{c,2}(\vec{q})}{e_{ref}} + w_{c,3} \frac{e_{c,3}^2(\vec{q})}{e_{ref}^2} + w_{c,4} \frac{e_{c,4}(\vec{q})}{e_{ref}} + w_{wa,1} \frac{e_{wa,1}^2(\vec{q})}{e_{ref}^2} + w_{wa,2} \frac{e_{wa,2}(\vec{q})}{e_{ref}}$$

$$+ w_{rm,1} \frac{e_{rm,1}^2(\vec{q})}{e_{ref}^2} + w_{rm,2} \frac{e_{rm,2}(\vec{q})}{e_{ref}} + w_{constraints} \frac{e_{constraints}^2(\vec{q})}{e_{ref}^2} \ \Big] \tag{28}$$

$$W = w_{es} + w_{m,1} + w_{m,2} + w_{p,1} + w_{p,1} + w_{p,3} + w_{p,4} + w_{e,1} + w_{c,1}$$
$$+ w_{c,2} + w_{c,3} + w_{c,4} + w_{wa,1} + w_{wa,2} + w_{rm,1} + w_{rm,2} + w_{constraints} \tag{29}$$

In the above equations, the following abbreviations have been used for the indices of the energy errors, $e_{type,n}$ or $e_{type}$: es = electrostatic potential, m = minimization data, p = packing data, c = conformation data, w = wide amplitude data, rm = reverse minimization data, constraints = constraint terms to be defined. The additional sub-type index, n, takes values from I to 4, which indicate energies at a potential energy minimum, forces at a potential energy minimum, energies around a minimum and forces around a minimum, respectively. The vector $\vec{q}$ represents the refinable force field parameters. Some of the terms in eq. 28 are proportional to the energy error, while others are proportional to its square route. This choice is explained in subsection 4.2. To combine both types of terms in a single expression, they are divided by a reference energy, $e_{ref}$, or the square of the reference energy, respectively. The reference energy, $e_{ref}$, can be interpreted as the target energy error per atom. A value of $e_{rel} = 0.01$ kcal/mol is in general appropriate and yields a value close to one for the deviation function after parameter refinement. The parameters $w_{type,n}$ or $w_{type}$ are user-defined weights. In principal, the deviation function is constructed in such a way that all weights can be set to 1, but we will see later on that there are valid reasons to reduce the importance of certain terms in some cases.

### 4.1 Electrostatic potential data

Using a test charge Q, the electrostatic potential differences between the tailor-made force field and the hybrid method can be easily converted to an average energy error, $\varepsilon_j$, for a crystal structure, j :

$$\varepsilon_j^2(\vec{q}) = \frac{\sum_i m_{i,j} Q^2 (\phi_{i,j}(\vec{q}) - \phi_{i,j,ref} - \phi_{offset,j})^2}{\sum_i m_{i,j}} \tag{30}$$

Here, $m_{i,j}$ is the multiplicity of each point i in the unit cell of structure j for which the electrostatic potential has been calculated with the hybrid method. $\phi_{offset,j}$ is a constant but unknown offset between the electrostatic potential values calculated with the two methods. $\phi_{offset,j}$ is chosen as to minimize $\varepsilon_j^2$. We typically use a test charge of Q=0.3e. In general, the overall electrostatic data term must take into account contributions from several crystal structures.

$$e_{ep}^2(\vec{q}) = \frac{\sum_j w_j n_{asym,j} \varepsilon_j^2(\vec{q})}{\sum_j w_j n_{asym,j}} \tag{31}$$

Here $w_j$ is an additional weight which is equal to 1.0 in most cases. The parameter $n_{asym}$ is the number of atoms per asymmetric unit.

We initially used eqs. 30 and 31 to evaluate the electrostatic contribution to the deviation function, but this choice turned out to be inconvenient. Indeed, even if all atomic charges are allowed to vary freely, $\varepsilon_j$ is much larger than $e_{ref}$ in most cases because point charges do not describe the electrostatic potential very well, and the electrostatic term tends to dominate the deviation function. We therefore have developed another indicator, $\tilde{\varepsilon}_j$:

$$\tilde{\varepsilon}_j^2 = e_{ref} \frac{\varepsilon_j^2 - \varepsilon_{min,j}^2}{e_{ref} + 2\varepsilon_{min,j}} \tag{32}$$

In the above equation, $\varepsilon_{min,j}$ is is the value that is obtained in for $\varepsilon_j$ if the atomic charges of structure j are fitted to the electrostatic potential without any additional constraints. For $\varepsilon_{min,j} \rightarrow 0$, i.e. if point charges would in principal allow for a perfect fit, we return to the intuitive choice $\tilde{\varepsilon}_j = \varepsilon_j$. If a parameter refinement is more or less converged, we typically observe $\varepsilon_j \approx \varepsilon_{j,min} \gg e_{ref}$ and eq. 32 reduces to

$$\tilde{\varepsilon}_j^2 \approx e_{ref}(\varepsilon_j - \varepsilon_{min,j}). \tag{33}$$

i.e. $\tilde{\varepsilon}_j^2$ is proportional to the additional misfit. Bond increments fitted to the electrostatic potential alone are not necessarily the best choice for the calculation of accurate lattice energies. For atoms participating in hydrogen bonds, for instance, it is more important that the combination of Coulomb and vdW interactions yields a balanced description of the energies of the different hydrogen bonds that can be formed. The question then is how far the bond increments are allowed to deviate from their ideal values with respect to the electrostatic potential, and the additional misfit captured by eqs. 32 and 33 provides an appropriate constraint. In eq. 31, $\varepsilon_j^2$ has to be replaced by $\tilde{\varepsilon}_j^2$.

### 4.2 Minimization data

Minimization data provide target values for lattice energies and energy gradients at local energy minima. The term $e_{m,l}$ in the deviation function compares lattice energies calculated with the tailor-made force field and the hybrid method:

$$e_{m,1}^2(\vec{q}) = \frac{1}{\sum_j w_j N_j} \sum_j w_j \sum_{i=0}^{N_j - 1} \left[ \frac{E_{ff,i,j}(\vec{q})}{n_{cell,i,j}} - \frac{E_{ref,i,j}}{n_{cell,i,j}} - e_{offset,j} \right]^2 \tag{34}$$

Index j runs over all polymorph sets used for the generation of minimization data, while index i covers all $N_j$ structures contained in a polymorph set j. $E_{ff,i,j}$ and $E_{ref,i,j}$ are the lattice energies calculated with the force field and the hybrid method, respectively, and $n_{cell,i,j}$ is the number of atoms per unit cell.

The energy offset $e_{offset,j}$ requires some special attention. For model systems consisting of the same molecular components, there is in general an unknown but constant offset between the energies calculated with a force field and those calculated with the hybrid method (or any pure DFT code). This offset can be traced back to the fact that the energy terms are not calibrated with respect to the energy that is released when atoms come together to form a molecule. For practical applications of force field calculations, this calibration is not required, because force field are used to compute energy differences between different molecular arrangements or conformations, whereby the molecules under consideration remain the same. Hence, the energy offset affects all states in the same way and drops out of the equation. In our parameter refinement, on the contrary, the offset needs to be taken into account explicitly.

For a computational point of view, the easiest choice would have been to adjust all offsets $e_{offset,j}$ independently as to minimize eq. 34. However, the use of too many adjustable offsets may result in a loss of essential information. Imagine a case where several crystal structures have been generated for each a molecule D with an h-bond donor, a molecule A with an h-bond acceptor and a pair of A and D. If the hydrogen bond is formed in all structures generated for the A - D pair and if the offset used for these structures is not related to the two offsets of the structures containing only A or D, the information about the h-bond formation energy is lost. To avoid this kind of problem, the offset for each polymorph set is calculated from molecular offsets:

$$e_{offset,j} = \frac{\sum_k M_{k,j} m_k e_{molecule,k}}{\sum_k M_{k,j} m_k} \qquad (35)$$

Here, $e_{molecule,k}$, is the energy offset per atom of molecule k, $m_k$ stands for the number of atoms in molecule k and $M_{k,j}$ is the number of molecules k in the repeat unit that is common to all structures of the polymorph set j. We handle energy offsets in a flexible way. If required, independent offsets can be used for certain polymorph sets, and different offsets can be defined for the rigid and the flexible version of a molecule. The same molecular offsets $e_{molecule,k}$ can be used for the similar terms $e^2_{m,1}$, $e^2_{p,1}$, $e^2_{c,1}$, $e^2_{wa,1}$ and $e^2_{rm,1}$. The molecular offsets are treated as free parameters which are adjusted at every evaluation of the deviation function such that it adopts the smallest possible value.

We now turn our attention to the term $e_{m,2}$ that deals with energy gradients. Each structure in a minimization data set corresponds to a potential energy minimum according to the hybrid method and a approximate Hessian Matrix H is available. In general, the energy minima of the tailor made force field and the hybrid method do not quite agree. At the minimum according to the hybrid method, the force field typically yields a non-zero gradient $\vec{g}_{ff}$, and a lattice energy minimization with the force field would yield an energy decrease by approximately

$$\Delta E = \frac{1}{2} \vec{g}_{ff}^T H^{-1} \vec{g}_{ff} . \qquad (36)$$

We use the expected energy change upon minimization to convert energy gradients to energy errors per atom and get:

$$e_{m,2}(\vec{q}) = \frac{1}{\sum_j w_j N_j} \sum_j w_j \sum_{i=0}^{N_j-1} \frac{\vec{g}_{ff,i,j}^T(\vec{q}) H_{i,j}^{-1} \vec{g}_{ff,i,j}(\vec{q})}{n_{cell,i,j}} \qquad (37)$$

Here the meaning of the sums and indices is the same as in eq. 34.

We now come back to an important point, namely the question why some terms in the deviation function, such as $e^2_{m,1}$, are squared, while others, such as $e_{m,2}$ are not. Let us assume that we have obtained a perfect match with the reference data, i.e. $e^2_{m,1}$ and $e_{m,2}$ are both zero. If we now change one force field parameter q by a small amount $\Delta q$, the lattice energies and energy gradients change by $(\partial E_{ff,i,j}/\partial q) * \Delta q$ and $(\partial \vec{g}_{ff,i,j}/\partial q) * \Delta q$, respectively. By construction, the increase of both $e^2_{m,1}$ and $e_{m,2}$ is proportional to the second power of $\Delta q$. If we would use $e_{m,1}$ instead of $e^2_{m,1}$ or $e^2_{m,2}$ instead of $e_{m,2}$, one of the terms would depend much stronger on $\Delta q$ than the other. This is undesirable if energies and gradients are to be taken into account in a balanced fashion. It is a general feature of our deviation function that terms dealing with energies are squared, while terms referring to forces are not.

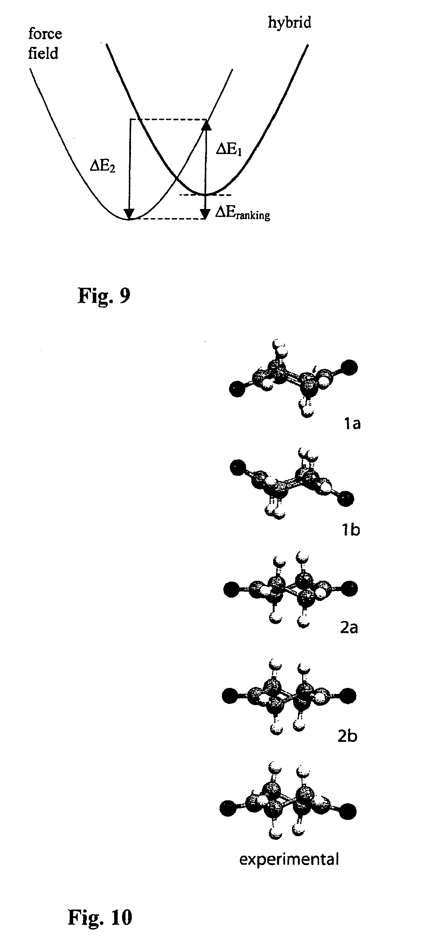

It is worthwhile to clarify the relationship between the terms $e^2_{m,1}$ and $e_{m,2}$ and the energy ranking of molecular crystals. The term $e^2_{m,1}$ captures the difference between lattice energies calculated with the tailor-made force field and the hybrid method, represented by $\Delta E_1$ in Fig. 9, for a set of fixed crystal structures which correspond to local minim according to the hybrid method. The term $e_{m,2}$ takes into account the energy shift of these structures upon optimization with the tailor-made force field, as indicated by $\Delta E_2$ in Fig. 9. The energy difference $\Delta E_{ranking} = \Delta E_1 + \Delta E_2$ between the lattice energies of one and the same crystal structure fully optimized with the hybrid method and the tailor-made force field, is not directly considered in the deviation function.

### 4.3 Packing data

The first two terms, $e^2_{p,1}$ and $e_{p,2}$ are calculated according to eqs. 34 and 37. For the third term, $e^2_{p,3}$, which takes into account the lattice energies calculated at points around the local minima, we follow a completely different road. The energies at surrounding points characterize the steepness of the potential energy well. In the context of lattice energy ranking, these energies are relevant when it comes to the calculation of a free energy correction. We have therefore chosen to relate the differences between the tailor-made force field and the reference data to some kind of free energy difference at room temperature. Before we continue, it is important to stress that we do not intend to develop a formula for the accurate calculation of free energy differences. Instead, our only aim is to include the energies calculated with the force field in the deviation function in a physically meaningful way.

Let us assume that the position of the local minimum is the same with the tailor-made force field and the hybrid method, whereby the two methods yield lattice energies $E_{ff,min}$ and $E_{ref,min}$, respectively. At a point v a distance $\Delta x_v$ away from the minimum, lattice energies $E_{ff,v}$ and $E_{ref,v}$ are obtained. The energy differences

$$\Delta E_{Y,v} = E_{Y,v} - E_{Y,min} \tag{38}$$

can be converted to force constants and angular frequencies

$$K_{Y,v} = 2\Delta E_{Y,v} / \Delta x_v^2 \tag{39}$$

and

$$\omega_{Y,v} = \sqrt{K_{Y,v} / m_v} \, , \tag{40}$$

whereby Y stands for 'ff' or 'ref' and $m_v$ is the effective mass of the displacement from the minimum to the point v in a multidimensional coordinate space. The free energy difference of two classical harmonic oscillators with angular frequencies $\omega_{ff,v}$ and $\omega_{ref,v}$ is given by

$$\Delta G_v = kT_{RT} \ln \frac{\omega_{ff,v}}{\omega_{ref,v}} = kT_{RT} \ln \sqrt{\frac{\Delta E_{ff,v}}{\Delta E_{ref,v}}} \, . \tag{41}$$

The singularity at $\Delta E_{ff,v} = 0$ makes the above expression inconvenient for direct use in the deviation function. Instead, we use first term of the Taylor expansion around $\Delta E_{ff,v} = \Delta E_{ref,v}$. Dividing by the number of atoms per unit cell, $n_{cell}$, and adding an additional factor ½ to reflect the fact that the point v corresponds to only 'half' a vibrational mode, we come to a free energy difference per atom,

$$\Delta g_v = kT_{RT} * \frac{\Delta E_{ff,v} - \Delta E_{ref,v}}{4\Delta E_{ref,v} n_{cell}} \, . \tag{42}$$

Assuming geometrical error propagation for the free energy errors $\Delta g_v$ associated with a single structure and summing over all polymorph sets, j, and the corresponding structures, i, we finally get

$$e_{p,3}^2(\vec{q}) = \frac{1}{\sum_j w_j N_j} \sum_j w_j \sum_{i=0}^{N_j-1} \sum_v \Delta g_{v,i,j}^2(\vec{q}) \tag{43}$$

It is difficult to derive a physically meaningful formula that takes into account the forces obtained at the points around the local minimum. We have therefore 'guessed' an appropriate term starting from $e^2_{p,3}$ in analogy to the change that leads from $e^2_{p,1}$ to $e_{p,2}$.

$$\Delta \widetilde{E}_{v,i,j}(\vec{q}) = \frac{1}{2}(g_{ff,v,i,j}(\vec{q}) - \bar{g}_{ref,v,i,j})^T \overline{H}_{i,j}^2 (g_{ref,v,i,j}(\vec{q}) - \bar{g}_{ref,v,i,j}) \tag{44}$$

$$\Delta \widetilde{g}_{v,i,j}(\vec{q}) = \left( \frac{kT}{4\Delta E_{hybrid,i}} \right)^2 \frac{\Delta \widetilde{E}_{v,i,j}(\vec{q})}{n_{cell}} \tag{45}$$

$$e_{p,4}(\vec{q}) = \frac{1}{\sum_j w_j N_j} \sum_j w_j \sum_{i=0}^{N_j-1} \sum_v \Delta \widetilde{g}_{v,i,j}(\vec{q}) \tag{46}$$

### 4.4 Expansion data

Expansion data are dealt with in very much the same way as the energies of minimization data:

$$e_{e,1}^2(\vec{q}) = \frac{1}{\sum_j w_j N_j} \sum_j w_j \sum_{i=0}^{N_i-1} \sum_{l=0}^{M_{i,j}-1} \frac{1}{M_{i,j}-1} \left[ \frac{E_{ff,l,i,j}(\vec{q})}{n_{cell,i,j}} - \frac{E_{ref,l,i,j}}{n_{cell,i,j}} - e_{offset,i,j} \right]^2 . \tag{47}$$

Compared to eq. 34, there is an additional sum over the $M_{i,j}$ states that take each crystal structure from the densely packed to the fully expanded state. The offset is now adjusted for every structure independently as to minimize eq. 47:

$$e_{offset,i,j} = \frac{1}{M_{i,j}} \sum_{l=0}^{M_{i,j}-1} \frac{E_{ff,l,i,j}(\vec{q})}{n_{cell,i,j}} - \frac{E_{ref,l,i,j}}{n_{cell,i,j}} \tag{48}$$

We have chosen to use independent offsets because we are only interested in the energy difference between the various states of expansion here. The densely packed state is in general also part of a minimization data or packing data set. Using an offset linked to other data sets would result in double counting.

### 4.5 Conformation data

The energy terms for conformation data are constructed in analogy to the terms for packing data. However, since the vibrational energy levels of intramolecular modes are typically of the order of or larger than $kT_{RT}$, we have to replace eq. 41 by the corresponding expression for the quantum mechanical harmonic oscillator. Following the way outlined in subsection 4.3, we arrive at expressions identical to eqs. 43 and 46 for the terms $e^2_{c,3}$ and $e_{c,4}$, but we have to replace eq. 42 by

$$\Delta g'_{v,i,j}(\vec{q}) = \frac{0.5 + \exp(-\dfrac{\hbar\omega_{ref,v,i,j}}{kT_{RT}})}{1 - \exp(-\dfrac{\hbar\omega_{ref,v,i,j}}{kT_{RT}})} \frac{\hbar\omega_{ref,v,i,j}}{4} \frac{\Delta E_{ff,v,i,j}(\vec{q}) - \Delta E_{ref,v,i,j}}{\Delta E_{ref,v,i,j} n_{cell}} \tag{49}$$

and eq. 45 by

$$\Delta \widetilde{g}'_{v,i,j}(\vec{q}) = \left( \frac{0.5 + \exp(-\dfrac{\hbar\omega_{ref,v,i,j}}{kT_{RT}})}{1 - \exp(-\dfrac{\hbar\omega_{ref,v,i,j}}{kT_{RT}})} \frac{\hbar\omega_{ref,v,i,j}}{4\Delta E_{ref,v,i,j}} \right)^2 \frac{\Delta \widetilde{E}_{v,i,j}(\vec{q})}{n_{cell}} . \tag{50}$$

### 4.6 Wide amplitude data

Wide amplitude data are integrated in the deviation function in exactly the same way as minimization data (see eqs. 34 and 37), whereby each sequence of frames forms a polymorph set. It is important to note that each frame corresponds to a stable structure because of the presence of additional constraint terms. The constraint terms contribute to all energies and energy derivatives, i.e. the energies and gradients calculated with the hybrid method, the Hessian matrices and the energies and gradients calculated with the tailor-made force field.

### 4.7 Reverse minimization data

The terms $e^2_{rm,1}$ and $e_{rm,2}$ are defined in analogy to the terms $e^2_{m,1}$ and $e_{m,2}$ in eqs. 34 and 37, whereby $\vec{g}_{ff,i,j}$ in eq. 37 has to be replaced by $(\widetilde{g}_{ff,i,j} - \widetilde{g}_{ref,i,j})$.

### 4.8 Parameter constraints

For reasons which will be outlined in section 5, we uses a special constraint term to impose that the references angles, $\theta_0$, of the angle bend terms (see eq. 14) found around certain central atoms match a geometrically possible atomic configuration. The overall constraint term is a weighted sum over individual constraint terms $c_{4,m}$ and $c_{3,m}$ for atoms with four covalently bonded neighbours and atoms with three covalently bonded neighbours in a planar configuration, respectively.

$$e^2_{constraints}(\vec{q}) = \frac{e^2_{ref}}{\alpha^2_{ref}} \frac{1}{N_4 + N_3} \left[ \sum_{m=0}^{N_4-1} c_{4,m}(\vec{q}) + \sum_{m=0}^{N_3-1} c_{3,m}(\vec{q}) \right] \tag{51}$$

The relative importance of the overall constraint term is adjusted via the parameter $\alpha_{ref}$, which we typically set to 0.1 °.

For three atoms j, k and l around a central atom i in a planar configuration with FFATs $t_i$, $t_j$, $t_k$ and $t_l$, the corresponding constraint is defined by

$$c_3 = (360° - \theta_{t_j t_i t_k} - \theta_{t_j t_i t_l} - \theta_{t_k t_i t_l})^2 . \tag{52}$$

For a central atom with 4 covalently bonded neighbours, the constraint term is not that simple. In total, there are 6 angle bend terms with reference angles $\theta_{i,0}$, whereby i now is an index that runs from 0 to 5 to simplify the notation. For a particular atomic arrangement, the covalent bonds form 6 angles $\alpha_i$, whereby each angle can be calculated from the other five angles:

$$\alpha_i = A(..., \alpha_{j \neq i}, ...) . \tag{53}$$

The function A can be derived from geometric considerations. We use the above relationship to construct a constraint term for the reference angles, $\theta_{i,0}$ :

$$c_4 = \frac{1}{6} \sum_{i=0}^{5} (\theta_{i,0} - A(..., \theta_{j \neq i}, ...))^2 . \tag{54}$$

**5. Parameter optimization algorithm**

In general, force field parameters are strongly coupled and an optimization algorithm for force field parameters refinement must be able to find its way through long and flat valleys in a parameter space of more than 100 dimensions. We use a two step procedure consisting of a Powell optimization and a quasi-Newton optimization with numerical first and second derivatives.

A mayor problem for force field parameter refinement are directions in the parameter space along which several force field parameters can changes substantially without having a significant impact on the value of the deviation function. The two possible origins of such soft directions are incompleteness of the reference data and redundant force field energy terms. It is frequently not possible to eliminate soft directions simply by the generation of more reference data or by the elimination of redundant energy terms, because the data generation may be too time consuming or because redundant energy terms allow for a mathematical structure of the force field that is convenient to use. Soft directions, if not dealt with appropriately, typically result in 'unphysical' force field parameters, i.e. values that deviate from commonly admitted parameter ranges for bond distances, angles, force constants, torsion barriers and others. Unphysical parameters resulting from true redundancies are in principle not a problem, since the sum of all energy terms should yield an appropriate description of the potential energy in this case. However, unphysical parameters may as well result from poor reference data or lack of convergence, in which case energy calculations for structures outside the training set may give disastrous results. Hence, physical reasonable parameter ranges should always be imposed as a safeguard against severe energy errors.

The notion of physically reasonable values is not limited to individual parameters, but should also be applied to parameter sequences and certain groups of parameters. We use different strategies to deal with these categories. For every individual force field parameter, we define a lower and an upper bound, whereby the bounds must be chosen generously to accommodate for the full range of admissible values. In cases where sequences of force field parameters are expected to exhibit a certain regularity, these parameters can be defined as a function of a reduced numbers of other parameters which are adjusted in the parameter refinement. For instance, one may impose that the homo-atomic vdW parameters $B_{ii}$ (see eq. 8) are a linear function of the number of valence electrons, $n_{val,i}$, for the elements C, N, O and F:

$$B_{i,i} = a + n_{val,i} * b , \tag{55}$$

where a and b are the adjustable parameters in the parameter refinement.

For certain groups of parameters, the relationships to be satisfied may be to complex to be dealt with by means of a user-defined function. One particular important example are the reference angles, $\theta_0$, of the angle bend terms around a common central atom with four covalently bonded neighbours. In this case, only 5 out of the 6 angles formed by the covalent bonds are required to describe the atomic arrangement around the central atom. If all six reference angles are allowed to vary independently, the reference angles frequently move away substantially from the ideal tetrahedral angle of 109.5°, giving rise to a situation where the overall geometry results from the equilibrium between 6 highly strained angle bend terms. Most of the strain can be avoided by forcing the six reference angles to take values that correspond to a geometrically possible atomic arrangement around the central atom, and we have added a special constraint term to the deviation function for this purpose as already discussed in subsection 4.8. Planar arrangements of a central atom with three covalently bonded neighbours are dealt with in a similar fashion, even though the use of a user-define function is a valid alternative in this case.

## 6. A recipe

In the previous sections, extensive details for specifying advantageous embodiments of the invention have been presented. We now summarize the overall procedure of an advantageous embodiment:

1. Build the molecule and molecular fragments, if required.
2. Generate force field atom types and molecular typing rules. The molecule and the fragments may share some FFATs.
3. Perform a conformer analysis of the molecule using a standard force field.
4. Optimize the geometries of all conformers (of the full molecule) and of the fragments with the hybrid method. If some conformers are identical after the optimization the duplicates have to be discarded. One may also discard energetically unfavourable conformations that have no chance to be observed experimentally.
5. Generate electrostatic data for all conformations and fragments.
6. Fit bond increments to the electrostatic data. The molecule and the fragments may share some bond increments.
7. Take vdW parameter from a standard force field or from a previous parameter refinement. In conjunction with the bond increments from step 6, you now have a rough force field suitable for rigid molecules.
8. Generate rigid molecule crystal packings for each fragment, each pair of fragments and selected conformations of the full molecule in all space groups with no more than two symmetry copies in the reduced unit cell.
9. Select an appropriate number of structures from the crystal packings generated in step 8 to cover all relevant close contacts.
10. Generate rigid body minimization data for all structures selected in step 9.
11. Generate rigid body minimization data under an appropriately chosen pressure for all or some structures selected in step 9.
12. Generate expansion data for some or all of the structures optimized in step 10.
13. Fit the vdW parameters to rigid body minimization data (steps 10 and 11) and expansion data (step12).
14. Fit the bond increments and vdW parameters to electrostatic data (step 5), rigid body minimization data (steps 10 and 11) and expansion data (step 12). Your tailor-made force field is now fully optimized for intermolecular interactions.
15. Generate conformation data for all molecular conformations.
16. Fit the intramolecular force field parameters to conformation data (step 15).
17. Generate wide amplitude data for all relevant conformational changes.
18. Fit the intramolecular force field parameters to conformation data (step 15) and wide amplitude data (step 17). Your tailor-made force field is now fully optimized for intra- and intermolecular interactions.
19. Perform a conformer analysis with the tailor-made force field. If you find more conformations then at the end of step 4, special action is required which will be discussed below.
20. Generate crystal structures in P1 or in all space groups with no more than 2 symmetry copies in the reduced unit cell, optimize the structures with the tailor-made force field and the hybrid method and compare the lattice energies. This step provides you with a first, rough estimate of the overall accuracy of the tailor-made force field.

If the conformer analysis in step 19 yields additional conformations, all new conformations have to be optimized with the hybrid method. If the hybrid method confirms that a new conformation corresponds to a local minimum of the potential energy hypersurface, additional conformation data and wide amplitude data must be generated accordingly. In the opposite case, reverse minimization data need to be generated to flag the new conformation as a false local minimum. After all new conformations have been dealt with, the procedure continues with step 18, taking in account the additional reference data as well as the conformations data and wide amplitude data already generated in steps 15 and 17. If a new conformation has been found that is very different from the conformation used previously for the generation of reference data, it may also we necessary to calculate electrostatic potential data, rigid body minimization data under ambient and high pressure and expansion data for the new conformation. In this case, the procedure continues with step 13.

It sometimes may be appropriate to feed the minimization data implicitly generated in step 20 back into the parameter refinement. In this case, all force field parameters are refined simultaneously in a last refinement step, taking into account all reference data.

## 7. Computer hardware

All calculations described in the subsequent two sections have been carried out on a self-build LINUX PC cluster of variable size, ranging from 1 to 16 nodes. Every node consisted among other components of a single PentiumIV processor running at 2.8 to 3.2 GHz, an ASUS P4P800-E motherboard, generic PC3200 memory sticks with a total capacity of 2 - 3 GB and a 40 GB hard disk. The nodes were connected via a now standard Gigabit Ethernet.

At the time of purchase in 2003 and 2004, the total price per node was about 700 Euros before tax. We mention this number in order to stress that the overall cost of our cluster is rather small compared to other laboratory equipment.

For cluster management, we used the ROCKS 4.0.0 cluster toolkit[26].

The time consuming parts of our calculations involve the optimization of tens or hundreds of crystal structures with the hybrid method. With respect to parallel computing, our tasks are of the embarrassingly parallel type, since it is sufficient to dispatch the structure optimization jobs over the available nodes, whereby each node deals with the optimization of a single structure independantly. Hence, the overall CPU time requirements scale about linearly with the number of nodes.

Strictly speaking, this is only true for crystal structures with reduced unit cell volumes up to approximately 1500 $Å^3$. Above, the memory requirements of the VASP DFT code exceed the amount of memory available on a single node with a 32bit operation system. In the range from 1500-3000 $Å^3$, we run VASP in parallel on 2 - 4 nodes. On the hardware described above, it is difficult to go beyond reduced unit cell volumes of 3000 $Å^3$. More than 2 nodes would be required to satisfy the overall memory requirements, but our system scales poorly beyond 2 nodes because the Gigabit Ethernet does not offer enough bandwidth. The practical limit for our approach clearly lies much higher than 3000 $Å^3$, since 64bit nodes with 10Gb Ethernet connections are nowadays readily available.

## 8. Case study: cyclohexane-1,4-dione

We have chosen cyclohexane-1,4,dione (CHD) as an example of a molecule with a flexible aromatic ring. In its category, CHD is particularly challenging, since the molecular conformation in the experimental crystal structure[27] measured at 133 K is distorted significantly with respect to the corresponding gas phase conformation, as shown in Fig. 10.

The force field atom types generated for CHD are shown in Fig. 2d. Since the molecule is rather small and contains at least two copies of every atom type, molecular fragments are not required.

Electrostatic data were generated for conformations 1a and 2a in Fig. 10. Rigid body minimization data at ambient pressure and 5 GPa as well as expansion data were prepared for 34 different crystal packings. These data were used to fit 13 parameters related to vdW interactions and 3 bond increments. A relatively small weight was used for the pressure data and the expansion data. Our simple non-bonded potentials do not allow for an accurate description of lattice energies over a large density range. Pressure data and expansion data are required to obtain a balanced parameter set, but must given a small weight to avoid a negative impact on the energy accuracy of the tailor-made force field with respect to lattice energy ranking at ambient pressure.

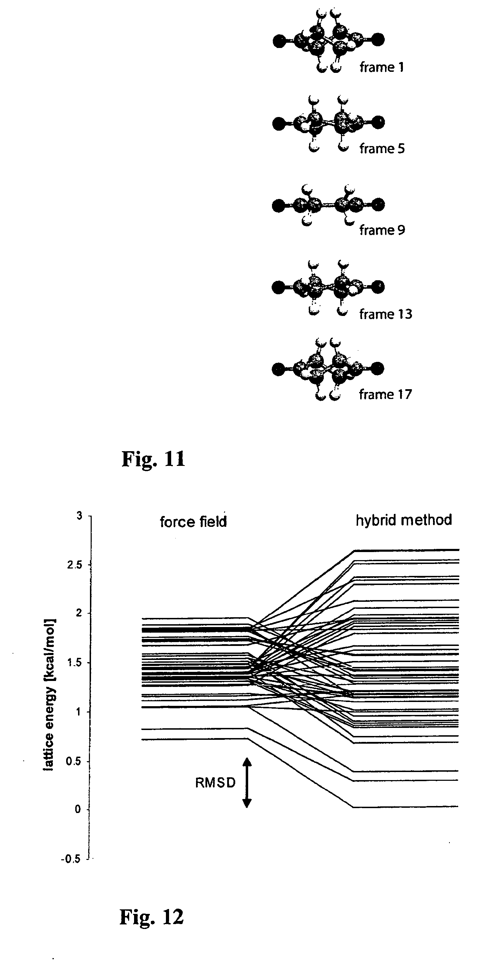

Conformation data were generated for conformers 1a and 2a, and wide amplitude data were produced for the transitions 1a->1b, 1a->2a and 2a->2b with 17 frames per transition. In order to illustrate what wide amplitude data look like, Fig. 11 shows 5 out of 17 frames for the transition 2a->2b. All molecules are contained in a simulation box with periodic boundary conditions which is not shown.

The conformation data and wide amplitude data were used to fit 8 bond stretch parameters, 12 angle bend parameters, 6 overall torsion parameters and 1 overall inversion parameters. Other intramolecular terms and parameters were not required.

With the tailor-made force field, crystal structures were first generated in space group P1 alone. The six resulting crystal structures were re-optimized with the hybrid method, resulting in root mean square deviation of 0.47 kcal/mol per molecule between the energy ranking with tailor-made force field and the hybrid method.

Then, a quasi-complete structure generation was carried out in all 230 space groups with one molecule per asymmetric unit, yielding 8 and 44 structures in an energy window of 0.5 kcal/mol and 1.0 kcal/mol, respectively. Our structure generation engine produces crystal structures which are fully optimized with the tailor-made force field. The most stable generated structure turned out to be the experimental structure.

The 57 most stable generated crystal structures were re-optimized with the hybrid method. Again, the experimental crystal structure was found as the most stable crystal structure. Fig. 12 compares the lattice energies obtained with the tailor-made force field and the hybrid method. On average, both methods differ by $\sigma_E$=0.53 kcal/mol per molecule.

**Table 2:** Comparison of the calculated and the experimental crystal structures for cyclohexane-1,4-dione. For each pair of structures, $\Delta a_{rel}$, $\Delta b_{rel}$, $\Delta c_{rel}$ and $\Delta \beta_{rel}$ are the relative changes of the lattice parameters. $\Delta$ is the unit cell deformation according to Ref. 4 and $\sigma_{\bar{r}}$ is the RMSD of the Cartesian coordinates of the non-hydrogen atoms.

| Structure 1 | Structure 2 | $\Delta a_{rel}$ [%] | $\Delta b_{rel}$[%] | $\Delta c_{rel}$[%] | $\Delta\beta_{rel}$[%] | $\Delta$[%] | $\sigma_{\bar{r}}$ [Å] |
|---|---|---|---|---|---|---|---|
| experimental | force field | -0.12 | -0.29 | -0.21 | 2.75 | 5.07 | 0.093 |
| experimental | hybrid method | 0.01 | 1.05 | 1.44 | 0.37 | 2.65 | 0.039 |
| force field | hybrid method | 0.12 | -0.76 | 1.64 | -2.38 | 5.16 | 0.101 |

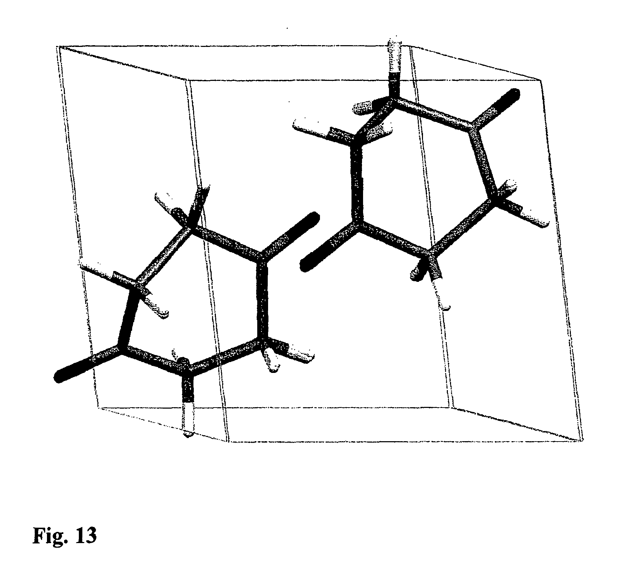

The experimental crystal structure and the corresponding structures that have been optimized with the tailor-made force field and the hybrid method are compared in Table 2. The experimental structure crystallized in space group P2$_1$, with unit cell parameters a = 6.65 Å, b = 6.21 Å, c = 6.87 Å and β = 99.82˚. Table 2 presents the relative differences of the lattice parameters, the unit cell deformation $\Delta$ defined in Ref. 4 and the root mean square deviation of the Cartesian coordinates of non-hydrogen atoms. According to the last two columns, which contain the most pertinent indicators, the result of the crystal structure optimization with the hybrid matches the experimental crystal structure fairly well, while the disagreements are about 2 times larger for the tailor-made force field. Fig. 13 shows a superposition of the experimental crystal structure and the result of a crystal structure optimization with the hybrid method. Both structures are so close that the difference is hardly noticeable, but some differences can be seen around the unit cell edges.

With respect to the main focus of this work, it is important to check if the recipe described in section 6 results in an overall accuracy close to the optimum within the limits of the chosen mathematical model. In order to investigate this issue, we have fed back 29 of the 57 crystal structures optimized with the hybrid method into the parameter refinement as minimization data. Minimization data for fully flexible crystal structures close to the global energy minimum can be expected to be the best possible data to parameterize a force field dedicated to crystal structure prediction. We have carried out two different parameter refinements. In one case (weak feedback), the additional minimization data were given approximately the same weight as the original rigid molecule minimization data. In a second refinement (strong feedback), the weight of the additional minimization data was increased by about a factor of 5 compared to all other data types.

After parameter refinement, the remaining 28 crystal structures not included in the refinement as minimization data were optimized with the two new variants of the tailor-made force field. For the force field according to our recipe and the two new variants, a comparison with the lattice energies from hybrid method for the 28 remaining structures yields root mean square deviations of 0.54 kcal/mol (recipe), 0.46 kcal/mol (weak feedback) and 0.40 kcal/mol (strong feedback). If the weight of the additional minimization data is increase even further, the energy error starts to increase again because the force field parameters are not sufficiently well defined by the additional minimization data alone. Compared to our recipe, even strong feedback reduced the energy error by only 26%. This is not a lot, taking into account that the recipe covers a large spectrum of different crystal packings, while the additional minimization data concentrate on the most stable crystal packings which certainly share a certain number of packing motifs. The 26% difference probably is the price to pay for an equilibrated coverage of a large spectrum of molecular arrangements. It has to be stressed that the above discussions are not of much practical importance anyhow, since the most stable crystal structures are not yet know when the tailor-made force field is parameterized initially. However, in some cases it may be appropriate to run two full crystal structure predictions, one with the initial tailor-made force field and one after feedback of the results from the first prediction into the parameter refinement. Such a procedure may increase the overall chance of finding the most stable crystal structure.

## References

(1) Lommerse, J. P. M. et al. Acta Crystallogr. B 2000, 56, 697.
(2) Motherwell, D. S. et al. Acta Crystallogr. B 2002, 58, 647.
(3) Day, G. M. et al. Acta Crystallogr. B 2005, 61, 511-527.
(4) Neumann, M. A.; Perrin, M.-A. J. Phys. Chem. B 2005, 109, 15531-15541

(5) Kresse, G.; Hafner, J. Phys. Rev. B 1993, 47, 558.

(6) Kresse, G.; Hafner, J. Phys. Rev. B 1994, 49, 14251.

(7) Kresse, G.; Furthmüller, J. Comput. Mat. Sci. 1996, 6, 15.

(8) Kresse, G.; Furthmüller, J. Phys. Rev. B 1996, 54, 11169.

(9) Kresse, G.; Joubert, D. Phys. Rev. B 1999, 59, 1758.

(10) The Polymorph Predictor and Cerius2 are products of Accelrys Inc., 9685 Scranton Road, San Diego, CA 92121-3752, USA.

(11) Neumann, M. A.; Perrin, M.-A.; Siebrecht, R.; Girard, P. to be submitted.

(12) Oganov, A. R.; Glass, C. W. J Chem. Phys 2006, 124, 244704-14

(13) Mayo, S. L.; Olafson, B. D.; Goddard III, W. A. J Phys. Chem. 1990, 94, 8897.

(14) MacKerrel, A. D. et al. J. Phys. Chem. B 1998, 102, 3586-3616

(15) Cornell, W. D et al. J. Am; Chem. Soc 1995, 117, 5179-5197

(16) Halgren, T. A. J Am. Chem. Soc. 1992, 114, 7827-7843

(17) Hagler, A. T.; Huler, E.; Lifson, S. J Am. Chem. Soc. 1974, 96, 5319.

(18) Sun, H. J. Phys. Chem. B 1998, 102, 7338-7364

(19) Rappé, A. K. et al. J Am. Chem. Soc. 1992, 114, 10024-10035

(20) Mooij, W. T. M; Leusen, F. J. J. Phys. Chem. Chem. Phys. 2001, 3, 5063-5066

(21) Day, G. M.; Motherwell, W. D.; Jones, W. Cryst. Growth Des. 2005, 5, 1023-1033

(22) Karamertzanis, P. G.; Price, S. L. J. Chem. Theory. Comput. 2006, 2, 1184-1199

(23) Brodersen, S. et al. Phys. Chem. Chem. Phys. 2003, 5, 4923-4931.

(24) Brodersen, S. et al. Cryst. Growth Des. 2005, 5, 925-933

(25) Halgren, T. A. J. Am. Chem. Soc. 1992, 114, 7827.

(26) Papadopoulos, P. M. et al Int. J. High Perf. Comp. Appl. 2004, 18, 317 - 326

(27) Mossel, A.; Romers, C. Acta Crystallogr. 1964, 17, 1217

(28) Andrew R. Leach: Molecular Modelling - Principles and Applications, Pearson Education Limited, ISBN 0-582-38210-6

**Claims**

**1.** A method for the prediction of crystal polymorphs for a given molecule, comprising the steps of:

   a) Parameterizing a force field for said molecule by fitting force field parameters to lattice energies and/or energy derivatives and/or electrostatic potential information generated with an accurate reference method;
   b) Generating a list of potential crystal structures for said molecule;
   c) For each of the generated structures, minimizing the lattice energy calculated with the force field by varying the atomic coordinates and the lattice parameters;
   d) Ranking the crystal structures according to their lattice energies as obtained in step c);
   e) Selecting some of the most stable crystal structures;
   f) For each of the selected structures, minimizing the lattice energy calculated with the accurate reference method by varying the atomic coordinates and the lattice parameters;
   g) Ranking the crystal structures according to their lattice energies as obtained in step f);
   h) Selecting a few crystal structures with the lowest lattice energies as candidates for the most stable experimental crystal structure.

**2.** A method for the determination of the parameters of a force field for the calculation of lattice energies and/or energy derivatives for at least one molecular crystal, comprising the steps of:

   A) Generating molecule specific force field atom types for at least one molecule;
   B) Producing at least one lookup table for mathematical functions and/or parameters, whereby the lookup table is indexed with respect to the abovementioned molecule specific atom types;
   C) Calculating reference data comprising lattice energies and/or energy derivatives and/or electrostatic potential information with an accurate reference method for at least one model system comprising atoms in a box with periodic boundary conditions;
   D) Calculating lattice energies and/or energy derivatives and/or electrostatic potential information using at least one mathematical function or one parameter from said at least one lookup table;
   E) Defining a deviation function (D) quantifying a total deviation between the lattice energies and/or energy derivatives and/or electrostatic potential information as calculated in steps C) and D), respectively;

F) fitting at least one parameter from said at least one lookup table in such a way as to minimize said deviation function (D).

3. A method according to claim 2, **characterized in that** the generation of molecule specific force field atom types in step A) comprises the following sub-steps:

A1) Identifying equivalent atoms in said molecule, whereby two atoms i and j in said molecule are considered to be equivalent if there is a permutation P that meets all conditions in a set of conditions, whereby the set contains at least the following conditions:

- i=P(j).
- The atoms k and P(k) belong to the same chemical element for all k.
- If two atoms, k and 1, are covalently bonded, so are the atoms P(k) and P(I), and vice versa;

A2) Assigning the same force field atom type to equivalent atoms.

4. A method according to any of claims 1 to 3, **characterized in that** the accurate reference method is a hybrid method that combines DFT calculations with an empirical van der Waals correction.

5. A method for the determination of lattice energies and/or energy derivatives of a crystal structure as a function of the atomic coordinates and the unit cell parameters, comprising the steps of:

α) Separating the total crystal energy into individual contributing energy terms;
β) Attributing force field atom types to all atoms in said crystal structure;
γ) Selecting appropriate parameters and a mathematical function for each energy term from look up tables indexed according to the force field atom types;
δ) Calculating the energy and/or the energy derivatives for each energy term;
ε) Summing over the energies and/or energy derivatives of the different energy terms.

6. A method according to claim 5, **characterized in that** at least one energy term is a function of an overall torsion angle defined as:

$$\varphi_{overall} = \frac{1}{AB} \sum_{a=0}^{A-1} \sum_{b=0}^{B-1} \left[ P_{]-180,180]} \left( \varphi_{i_a jkl_b} + \frac{360°}{A} a - \frac{360°}{B} b - \varphi_{i_0 jkl_0} \right) + \varphi_{i_0 jkl_0} \right]$$

whereby

- the atoms j and k, the atoms $i_a$ and j, and the atoms $l_b$ and k are respectively covalently bonded;
- A is the number of external neighbours of atom j;
- B is the number of external neighbours of atom k;
- The torsion angles $\varphi_{ijkl}$ measure the signed angle between the i-j-k plane and the j-k-l plane;
- $P_{]-180,180]}$ is a function that adds a multiple of 360° to its argument such that the result falls into the interval ]-180°,180°].

7. A method according to claim 5 or 6, **characterized in that** at least one energy term is a function of the signed center to plane distance

$$d_{ijkl} = \left( \vec{r}_l - \vec{r}_i \right) \cdot \frac{\left( \vec{r}_k - \vec{r}_i \right) \times \left( \vec{r}_j - \vec{r}_i \right)}{\left| \left( \vec{r}_k - \vec{r}_i \right) \times \left( \vec{r}_j - \vec{r}_i \right) \right|}$$

whereby

- The atom i is covalently bonded to exactly three atoms j, k, l.

- $\vec{r_i}$, $\vec{r_j}$, $\vec{r_k}$, $r_l$ are the Cartesian coordinates of the atoms i, j, k, l.

8. A method according to claim 7 **characterized in that** at least one energy term is given by

$$E_{term} = E_{inv}(d) + \frac{1}{2}k_1(d)(\alpha_1 - \alpha_{1,eq}(d))^2 + \frac{1}{2}k_2(d)(\alpha_2 - \alpha_{2,eq}(d))^2 + \frac{1}{2}k_3(d)(\alpha_3 - \alpha_{3,eq}(d))^2$$

$$\alpha_{3,eq}(d) = 360° - \alpha_{1,eq}(d) - \alpha_{2,eq}(d)$$

whereby the terms $E_{inv}(d)$, $k_1(d)$, $k_2(d)$, $k_3(d)$, $\alpha_{1,eq}(d)$ and $\alpha_{2,eq}(d)$ are defined as a truncated Taylor series of the distance $d = d_{ijkl}$, and the angles $\alpha_1$, $\alpha_2$ and $\alpha_3$ are obtained by projection of the bonds i-j, i-k and i-1 onto the j-k-1 plane.

9. A method according to any of claims 5 to 8, **characterized in that** the energy terms for the intramolecular Coulomb and/or van der Waals interactions of two atoms i and j are multiplied with a scale factor from a lookup table having entries that comprise at least one or more scale factors and a list of force field atom types that may contain wildcards, if the atoms i and j are connected via a chain of covalently bonded atoms including the terminal atoms i and j, whose force field atom types match one of the entries of the lookup table.

10. A method according to any of claims 5 to 9, **characterized in that** the attribution of force field atom types in step (β) involves at least one molecular typing rule, i.e. a set of force field atom types and a set of conditions, whereby:

- the force field atom types are attributed to the atoms in a molecule if it fulfills all specified conditions;
- For chiral molecules, at least one of the conditions of a molecular typing rule can only be fulfilled either by the molecule or by its mirror copy;
- The definition of a molecular typing rule for a chiral molecule as a so-called straight typing rule entails implicitly the definition of an 'inverse' molecule typing rule for its mirror copy;
- The use in an energy term of at least one parameter or one mathematical function from a lookup table depends on whether the atoms involved in this energy term were assigned force field atom types with the straight or the inverse typing rule.

11. A computer program product comprising computer readable code for enabling a computer to perform a method according to any of the preceding claims when said code is executed.

structure
generation

final
ranking

starting
Hessian

Force
field

parameterisation

Hybrid
method

**Fig. 1**

a

b

c

d

Fig. 2

Fig. 3

$2\Delta q_O$    $3\Delta q_H$

Fig. 4

Fig. 5

**Fig. 6**

**Fig. 7**

Fig. 8

Fig. 9

Fig. 10

frame 1

frame 5

frame 9

frame 13

frame 17

**Fig. 11**

**Fig. 12**

**Fig. 13**

**EUROPEAN SEARCH REPORT**

Application Number

EP 06 29 1901

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 577 822 A (AVANT GARDE MATERIAL SIMULATIO [FR]) 21 September 2005 (2005-09-21) | 1,4,11 | INV. G06F19/00 |
| Y | * paragraphs [0001], [0012], [0037] - [0057], [0250] * * table 5.6.a * | 5 | |
| X | US 2003/195734 A1 (SUN HUAI [US]) 16 October 2003 (2003-10-16) | 2-4,11 | |
| Y | * abstract * * paragraph [0003] - paragraph [0008] * | 5 | |
| D,X | NEUMANN M A ET AL: "Energy ranking of molecular crystals using density functional theory calculations and an empirical van der Waals correction" JOURNAL OF PHYSICAL CHEMISTRY B ACS USA, vol. 109, no. 32, 18 August 2005 (2005-08-18), pages 15531-15541, XP002462353 ISSN: 1089-5647 | 1,4,11 | |
| Y | * the whole document * | 5 | TECHNICAL FIELDS SEARCHED (IPC) G06F |

The present search report has been drawn up for all claims

| Place of search | Date of completion of the search | Examiner |
|---|---|---|
| The Hague | 13 December 2007 | Godzina, Przemyslaw |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document

EPO FORM 1503 03.82 (P04C01)

Europäisches
Patentamt
European
Patent Office
Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1,4,11

    A method and computer program product for the prediction of crystal polymorphs.

1.1. claims: 2-4,11

    A method and computer program product for the determination of the parameters of a force field.

1.2. claims: 5-11

    A method and computer program product for the determination of lattice energies and/or energy derivatives of a crystal structure.

    ---

Please note that all inventions mentioned under item 1, although not necessarily linked by a common inventive concept, could be searched without effort justifying an additional fee.

## ANNEX TO THE EUROPEAN SEARCH REPORT
## ON EUROPEAN PATENT APPLICATION NO.

EP 06 29 1901

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2007

| Patent document cited in search report | | Publication date | Patent family member(s) | | Publication date |
|---|---|---|---|---|---|
| EP 1577822 | A | 21-09-2005 | WO | 2005091178 A2 | 29-09-2005 |
| | | | US | 2007185695 A1 | 09-08-2007 |
| US 2003195734 | A1 | 16-10-2003 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LOMMERSE, J. P. M. et al.** *Acta Crystallogr. B,* 2000, vol. 56, 697
- **MOTHERWELL, D. S. et al.** *Acta Crystallogr. B,* 2002, vol. 58, 647
- **DAY, G. M. et al.** *Acta Crystallogr. B,* 2005, vol. 61, 511-527
- **NEUMANN, M. A ; PERRIN, M.-A.** *J. Phys. Chem. B,* 2005, vol. 109, 15531-15541
- **KRESSE, G. ; HAFNER, J.** *Phys. Rev. B,* 1993, vol. 47, 558
- **KRESSE, G. ; HAFNER, J.** *Phys. Rev. B,* 1994, vol. 49, 14251
- **KRESSE, G. ; FURTHMÜLLER, J.** *Comput. Mat. Sci.,* 1996, vol. 6, 15
- **KRESSE, G. ; FURTHMÜLLER, J.** *Phys. Rev. B,* 1996, vol. 54, 11169
- **KRESSE, G. ; JOUBERT, D.** *Phys. Rev. B,* 1999, vol. 59, 1758
- **OGANOV, A. R. ; GLASS, C. W.** *J Chem. Phys,* 2006, vol. 124, 244704-14
- **MAYO, S. L. ; OLAFSON, B. D. ; GODDARD III, W. A.** *J Phys. Chem.,* 1990, vol. 94, 8897
- **MACKERREL, A. D. et al.** *J. Phys. Chem. B,* 1998, vol. 102, 3586-3616
- **CORNELL, W. D et al.** *J. Am; Chem. Soc,* 1995, vol. 117, 5179-5197
- **HALGREN, T. A.** *J Am. Chem. Soc.,* 1992, vol. 114, 7827-7843
- **HAGLER, A. T. ; HULER, E. ; LIFSON, S.** *J Am. Chem. Soc.,* 1974, vol. 96, 5319
- **SUN, H. J.** *Phys. Chem. B,* 1998, vol. 102, 7338-7364
- **RAPPÉ, A. K. et al.** *J Am. Chem. Soc.,* 1992, vol. 114, 10024-10035
- **MOOIJ, W. T. M ; LEUSEN, F. J. J.** *Phys. Chem. Chem. Phys.,* 2001, vol. 3, 5063-5066
- **DAY, G. M. ; MOTHERWELL, W. D. ; JONES, W.** *Cryst. Growth Des,* 2005, vol. 5, 1023-1033
- **KARAMERTZANIS, P. G. ; PRICE, S. L. J.** *Chem. Theory. Comput.,* 2006, vol. 2, 1184-1199
- **BRODERSEN, S. et al.** *Phys. Chem. Chem. Phys.,* 2003, vol. 5, 4923-4931
- **BRODERSEN, S. et al.** *Cryst. Growth Des,* 2005, vol. 5, 925-933
- **HALGREN, T. A.** *J. Am. Chem. Soc.,* 1992, vol. 114, 7827
- **PAPADOPOULOS, P. M. et al.** *Int. J. High Perf. Comp. Appl.,* 2004, 317-326
- **MOSSEL, A. ; ROMERS, C.** *Acta Crystallogr.,* 1964, vol. 17, 1217
- **ANDREW R. LEACH.** Molecular Modelling - Principles and Applications. Pearson Education Limited